# EUROPEAN PATENT SPECIFICATION

(11) **EP 2 635 254 B1**
(45) Date of publication and mention of the grant of the patent: **15.05.2019**
(21) Application number: 11838873.5
(22) Date of filing: 04.11.2011
(51) Int. Cl.: A61K 8/90, A61K 8/67, A61K 9/14, A61K 9/56, A61K 31/355, A61K 31/765, A61K 47/30, A61K 9/48

(54) **COMPOSITIONS AND METHODS RELATING TO REDUCED MUCOADHESION**
ZUSAMMENSETZUNGEN UND VERFAHREN IM ZUSAMMENHANG MIT VERMINDERTER SCHLEIMHAUTADHÄSION
COMPOSITIONS ET PROCÉDÉS VISANT À UNE RÉDUCTION DE LA MUCOADHÉRENCE

(30) Priority: 05.11.2010 US 410539 P
(43) Date of publication of application: 11.09.2013
(73) Proprietor: The John Hopkins University, Baltimore, MD 21201 (US)
(72) Inventor: LAI, Samuel, K., Carrboro NC 27510 (US); YANG, Ming, Towson, MD 21286 (US); WANG, Ying-Ying, Baltimore, MD 21218 (US); MERT, Olcay, 06610 Ankara (TR); ENSIGN, Laura, Towson, MD 21204 (US); HANES, Justin, Baltimore, MD 21212 (US); FU, Jie, Baltimore, MD 21239 (US)
(74) Representative: Vossius & Partner Patentanwälte Rechtsanwälte mbB
(86) International application number: PCT/US2011/059321
(87) International publication number: WO 2012/061703

(56) References cited:
- US-A- 4 904 479
- US-A1- 2008 102 128
- US-A1- 2009 155 182
- US-A1- 2010 215 580

## Description

### Field of the Invention

The present invention is defined by the claims and generally relates to methods for reducing the mucoadhesive properties of a composition (*e*.*g*., a particle) and compositions having reduced mucoadhesive properties.

### Background of the Invention

Mucus is a viscoelastic and adhesive substance that traps most foreign particles (*e*.*g*., conventional drug and gene carriers) and helps protects certain body surfaces, for example, the respiratory, gastrointestinal, and cervicovaginal tracts and eyes (see, for example, Lai et al., Proc Natl Acad Sci, 2007, 104(5), 1482-7; Cone et al., Adv Drug Deliv Rev, 2009, 61(2), 75-85; Lai et al., Adv Drug Deliv Rev, 2009, 61(2), 158-71; Lai et al., Adv Drug Deliv Rev, 2009, 61(2), 86-100). The efficient trapping and removal of particles composed of FDA-approved polymers such as poly(lactide-co-glycolide) (PLGA) and poly(ε-caprolactone) (PCL) has strongly limited their use to treat or cure diseases of mucosal surfaces. Trapped particles cannot reach the underlying epithelium, and/or are quickly eliminated by mucus clearance mechanisms that occur on the order of minutes to hours (see, for example, Lai et al., Adv Drug Deliv Rev, 2009. 61(2), 158-71; Knowles et al., J Clin Invest, 2002. 109(5), 571-7). Thus, for sustained and/or targeted drug/gene delivery to epithelial cells, synthetic carrier particles must rapidly penetrate mucus secretions (see, for example, Lai et al., Adv Drug Deliv Rev, 2009, 61(2); Lai et al., Proc Natl Acad Sci, 2007, 104(5), 1482-7). To avoid rapid clearance, particles (*e*.*g*., comprising bioactive agents) must quickly penetrate viscoelastic and adhesive mucus gels following administration to mucosal tissues, a long-standing challenge in the field of drug delivery.

Mucus-penetrating particles (MPP) can be engineered by carefully tuning the surface properties of particles (see, for example, Lai et al., Adv Drug Deliv Rev, 2009, 61(2), 158-71). For example, a dense covalent coating of low molecular weight (MW) poly(ethylene glycol) (PEG) on surfactant-free polystyrene (latex) particles (PS-PEG) has been found to effectively reduce their affinity to mucus constituents (see, for example, Lai et al., Proc Natl Acad Sci, 2007, 104(5), 1482-7; Wang et al., Angew Chem Int Ed Engl, 2008, 47(50), 9726-9). This enables particles to diffuse rapidly in the interstitial fluid between mucus mesh fibers, without experiencing the bulk viscosity of mucus (see, for example, Lai et al., PLoS ONE, 2009, 4(1), e4294; Lai et al., Proc Natl Acad Sci, 107(2), 598-603), thereby enabling particles to diffuse across mucus at rates up to only 4-fold slower than those in water (see, for example, Lai et al., Proc Natl Acad Sci, 2007, 104(5), 1482-7; Wang et al., Angew Chem Int Ed Engl, 2008, 47(50), 9726-9). Moreover, US 2010/215580 describes a particle comprising an outer surface and one or more surface-altering moieties disposed on the outer surface that reduce mucoadhesion of the particle, wherein said particle diffuses through human cervicovaginal mucus at a diffusivity that is greater than 1/1000 the diffusivity that the particle diffuses through water at a time scale of 1 s.

However, to date, no system composed entirely of GRAS (Generally Regarded As Safe) components has been shown capable of penetrating human mucus. There are relatively few synthetic biodegradable polymers that have a history of safe use in humans and that can facilitate the encapsulation and controlled release of therapeutic agents. Two of the most prominent polymers are PLGA (used in various biomedical applications, including the Lupron Depot®, microspheres releasing leuprolide acetate to treat advanced prostate cancer (see, for example, Pillai et al., Curr Opin Chem Biol, 2001, 5(4), 447-51.), and PCL (used in adhesion barriers, sutures and orthopedic devices (see, for example, Kim et al., J Clin Periodontol, 2004.,31(4), 286-92)). Particles composed of these polymers provide important platforms for achieving sustained and/or targeted delivery of drugs and genes (see, for example, Wnek et al., Encyclopedia of biomaterials and biomedical engineering, 2004, New York, Marcel Dekker, Inc.). However, their use in drug delivery applications at mucosal surfaces has been severely limited by the protective mucus barrier coating these surfaces as PLGA and PCL are hydrophobic, causing particles composed of these materials to become immobilized in mucus due to polyvalent hydrophobic adhesive interactions with mucus constituents. This flaw has greatly hindered the development of synthetic drug carriers for the treatment of diseases of mucosal origin.

In addition, lack of stability of the particles for delivery to mucosal tissues presents challenges. To stabilize emulsions, inhibit coalescence, and reduce particle aggregation during particle synthesis, the surfaces of drug-loaded polymeric particles are usually coated with surfactants. Surfactants can also influence particle size, morphology, encapsulation efficiency, and drug release kinetics. A particular challenge in formulating drug-loaded MPP is that many commonly used surfactants either (1) yield mucoadhesive particles or (2) fail to facilitate efficient drug encapsulation. For example, poly(vinyl alcohol) (PVA) is one of the most widely used surfactants (see, for example, Shakesheff et al., J Colloid Interface Sci, 1997, 185(2), 538-47), but PVA-coated particles are strongly mucoadhesive, presumably due to strong hydrogen bonding between hydroxyl groups extending from the polymer backbone and mucin glycoproteins (see, for example, Peppas et al., European Journal of Pharmaceutics and Biopharmaceutics, 1997, 43(1), 51-58). Similarly, chitosan-coatings are also well established to result in strong mucoadhesion, presumably due to a combination of electrostatic attraction, hydrogen bonding, and hydrophobic effects (see, for example, Prego et al., Expert Opin Drug Deliv, 2005, 2(5), p. 843-54).

Accordingly, improved methods, compositions, and systems are needed for reducing the mucoadhesive properties of drug delivery devices.

### Summary

The present invention is defined by the claims. Hence the present invention provides a method of reducing mucoadhesion of a particle, wherein the particle comprises a poly(ethylene glycol)-vitamin E conjugate associated with at least a portion of the particle, the method comprising the steps of: associating a (poly(ethylene glycol))-(poly(propylene oxide))-(poly(ethylene glycol)) triblock copolymer with the surface of the particle, thereby forming a coated particle, wherein the molecular weight of the poly(ethylene glycol) of the poly(ethylene glycol)-vitamin E conjugate is greater than about 2 kDa, wherein the molecular weight of the (poly(propylene oxide)) block of the triblock copolymer is greater than about 1.8 kDa, and wherein said coated particle diffuses through human cervicovaginal mucus at a diffusivity that is less than 1/500 the diffusivity that the particle diffuses through water. The present invention furthermore relates to a particle comprising: a poly(ethylene glycol)-vitamin E conjugate; and a (poly(ethylene glycol))-(poly(propylene oxide))-(poly(ethylene glycol)) triblock copolymer coating, wherein the molecular weight of the poly(ethylene glycol) of the poly(ethylene glycol)-vitamin E conjugate is greater than about 2 kDa, wherein the molecular weight of the (poly(propylene oxide)) block of the triblock copolymer is greater than about 1.8 kDa as well as a pharmaceutical composition comprising a plurality of such particles and one or more pharmaceutically acceptable excipients. Generally described herein are methods for reducing mucoadhesion of a composition (*e*.*g*., a particle) and compositions having reduced mucoadhesion. Such compositions and methods can facilitate the movement of the composition through mucosal tissues. For example, a composition may comprise a plurality of particles having surface-altering agents which reduce the mucoadhesion of the particles, thus allowing for rapid diffusion of the particles through mucosal tissues. In some cases, a particle may comprise at least one bioactive agent and may be used for treating, preventing, and/or diagnosing a condition in a subject. A pharmaceutical composition may be well-suited for administration routes involving the particles passing through a mucosal barrier.

Also described herein is a method of forming a (poly(ethylene glycol))-(poly(propylene oxide))-(poly(ethylene glycol))-coated particle, the method comprising the steps of preparing a particle using a poly(ethylene glycol)-vitamin E conjugate (*e*.*g*., as a surfactant) and coating the particle with a (poly(ethylene glycol))-(poly(propylene oxide))-(poly(ethylene glycol)) triblock copolymer. The (poly(ethylene glycol))-(poly(propylene oxide))-(poly(ethylene glycol)) triblock copolymer may associate with the coated particle to form a (poly(ethylene glycol))-(poly(propylene oxide))-(poly(ethylene glycol))-coated particle.

Also described herein is a method of reducing mucoadhesion of a particle which comprises the steps of associating a (poly(ethylene glycol))-(poly(propylene oxide))-(poly(ethylene glycol)) triblock copolymer with the surface of the particle.

Also compositions are described herein. A composition may comprise a particle comprising one or more surface-altering moieties disposed on the surface of the particle that reduce mucoadhension of the particle, wherein the particle can be formed using a poly(ethylene glycol)-vitamin E conjugate, followed by coating the particle with a (poly(ethylene glycol))-(poly(propylene oxide))-(poly(ethylene glycol)) triblock copolymer. The molecular weight of the poly(ethylene glycol) of the poly(ethylene glycol)-vitamin E may be greater than about 2 kDa. The molecular weight of the (poly(propylene oxide)) block of the triblock copolymer may also be at least about 1.8 kDa.

Also a particle is described herein. A particle may comprise a polymeric core and a (poly(ethylene glycol))-(poly(propylene oxide))-(poly(ethylene glycol)) triblock copolymer associated with the surface of the polymeric core. Also a particle is described herein, wherein the particles is made using poly(ethylene glycol)-vitamin E conjugate, with a (poly(ethylene glycol))-(poly(propylene oxide))-(poly(ethylene glycol)) triblock copolymer.

The molecular weight of the (poly(propylene oxide)) block of the triblock copolymer utilized herein may be between about 1.8 kDa and about 10 kDa, or between about 2 kDa and about 10 kDa, or between about 3 kDa and about 10 kDa, or between about 4 kDa and about 10 kDa, or between about 1.8 kDa and about 5 kDa, or between about 3 kDa and about 5 kDa, or between about 2 kDa and about 4 kDa, or between about 2 kDa and about 5 kDa. The molecular weight of the (poly(propylene oxide)) block of the triblock copolymer may be at least about 1.8 kDa, or at least about 2 kDa, or at least about 2.5 kDa, or at least about 3 kDa, or at least about 4 kDa, or at least about 5 kDa. The molecular weight greater of the poly(ethylene glycol) of the poly(ethylene glycol)-vitamin E may be greater than about 2 kDa. The molecular weight of the (poly(propylene oxide)) block of the triblock copolymer may be greater than about 1.8 kDa. The molecular weight of the poly(ethylene glycol) of the poly(ethylene glycol)-vitamin E conjugate is typically between about 2 kDa and about 8 kDa, or between about 3 kDa and about 7 kDa, or between about 4 kDa and about 6 kDa, or between about 4.5 kDa and about 6.5 kDa, or about 5 kDa. In some cases, the poly(ethylene glycol)-vitamin E conjugate acts a surfactant.

A particle described herein may comprise surface-altering moieties disposed on the surface of the particle. The surface-altering moieties may be regions of the (poly(ethylene glycol))-(poly(propylene oxide))-(poly(ethylene glycol)) triblock copolymer localized on the surface of the particle. In the case of the (poly(ethylene glycol))-(poly(propylene oxide))-(poly(ethylene glycol)) triblock copolymer, each polymer molecule includes two surface-altering moieties (*i*.*e*., the poly(ethylene glycol) units). The surface-altering moieties may be present on the surface of the particles at a density between about 0.1 and about 10, or between about 0.1 and about 5, or between about 0.5 and about 5, or between about 0.1 and about 3, or between about 1 and about 10, or between about 0.5 and about 3, or between about 0.9 and about 2.8 surface-altering moieties per nm².

In some cases, a particle utilized herein diffuses through mucosal tissues (*e*.*g*., human cervicovaginal mucus) at a diffusivity that is less than approximately 1/500 the diffusivity that the particle diffuses through water on a time scale of approximately 1 second.

A particle utilized herein may be larger than about 1 nm, or about 5 nm, or about 20 nm, or about 100 nm, or about 200 nm, or about 500 nm in diameter. A particle may be formed using commonly known methods, for example, by nanoprecipitation. In some cases, nanoprecipitation comprises adding a solution of the particle material to a solvent in which the particle material is substantially insoluble. A particle may be coated with the (poly(ethylene glycol))-(poly(propylene oxide))-(poly(ethylene glycol)) triblock copolymer by exposing the particle to a solution comprising the (poly(ethylene glycol))-(poly(propylene oxide))-(poly(ethylene glycol)) triblock copolymer.

A particle may comprise a polymeric material (*e*.*g*., as a polymeric core). In some cases, the polymeric material is selected from the group consisting of polyamines, polyethers, polyamides, polyesters, polycarbamates, polyureas, polycarbonates, poly(styrenes), polyimides, polysulfones, polyurethanes, polyacetylenes, polyethylenes, polyethyeneimines, polyisocyanates, polyacrylates, polymethacrylates, polyacrylonitriles, and polyarylates. The polymeric material may be biodegradable and/or biocompatible. In some cases, the particle comprises a hydrophobic material and at least one bioactive agent. The hydrophobic material may be used instead of a polymer. The hydrophobic material may be used in addition to a polymer.

A particle typically comprises at least one bioactive agent. The particle may comprise at least two bioactive agents, or more. The bioactive agent can be encapsulated in the particle and/or disposed on the surface of the particle. The bioactive agent may or may not be covalently coupled to the particle. The bioactive agent may be an imaging agent, diagnostic agent, prophylatic agent, or therapeutic agent. The bioactive agent may be a nucleic acid, nucleic acid analog, small molecule, peptidomimetic, protein, peptide, lipid, carbohydrate, or surfactant.

The polymeric core may comprise a polymeric material and/or the particle may comprise a polymeric material selected from the group consisting of polyamines, polyethers, polyamides, polyesters, polycarbamates, polyureas, polycarbonates, polystyrenes, polyimides, polysulfones, polyurethanes, polyacetylenes, polyethylenes, polyethyeneimines, polyisocyanates, polyacrylates, polymethacrylates, polyacrylonitriles, and polyarylates. In some cases, the polymeric material is biodegradable and/or biocompatible.

The molecular weight of the (poly(propylene oxide)) block of the triblock copolymer comprised in the particle may be between about 1.8 kDa and about 10 kDa, or between about 2 kDa and about 10 kDa, or between about 3 kDa and about 10 kDa, or between about 4 kDa and about 10 kDa, between about 1.8 kDa and about 5 kDa, or between about 3 kDa and about 5 kDa, or between about 2 kDa and about 4 kDa, or between about 2 kDa and about 5 kDa. In some cases, the molecular weight of the (poly(propylene oxide)) block of the triblock copolymer is at least about 1.8 kDa, or at least about 2 kDa, or at least about 2.5 kDa, or at least about 3 kDa, or at least about 4 kDa, or at least about 5 kDa. The molecular weight of the poly(ethylene glycol) of the poly(ethylene glycol)-vitamin E conjugate comprised in the particle may be between about 2 kDa and about 8 kDa, or between about 3 kDa and about 7 kDa, or between about 4 kDa and about 6 kDa, or between about 4.5 kDa and about 6.5 kDa, or about 5 kDa.

A particle utilized herein may further comprise at least one bioactive agent. The bioactive agent may be encapsulated in the particle and/or disposed on the surface of the particle. The bioactive agent may or might not be covalently coupled to the particle. In some cases, the at least one bioactive agent is selected from the group consisting of imaging agents, diagnostic agents, therapeutic agents, agents with a detectable label, nucleic acids, nucleic acid analogs, small molecules, peptidomimetics, proteins, peptides, lipids, or surfactants.

A composition (e.g., a pharmaceutical composition) is also provided herein comprising at least one particle as described herein and at least one pharmaceutically acceptable excipients. The compositions may be used for treating, preventing, or diagnosing a condition in a patient. The treating, preventing, or diagnosing may comprise administering to a patient the composition. The composition may be administered to a mucosal tissue in the patient. In some cases, the composition is administered topically to the mucosal tissue in the patient.

Methods are provided comprising administering to a subject at least one particle and a (poly(ethylene glycol))-(poly(propylene oxide))-(poly(ethylene glycol)) triblock copolymer. In some cases, the molecular weight of the (poly(propylene oxide)) block of the triblock copolymer is greater than about 1.8 kDa.

### Brief Description of the Drawings

Other aspects will become apparent from the following detailed description when considered in conjunction with the accompanying drawings. The accompanying figures are schematic and are not intended to be drawn to scale. For purposes of clarity, not every component is labeled in every figure, nor is every component of each embodiment of the invention shown where illustration is not necessary to allow those of ordinary skill in the art to understand the invention. In case of conflict, the present specification, including definitions, will control.

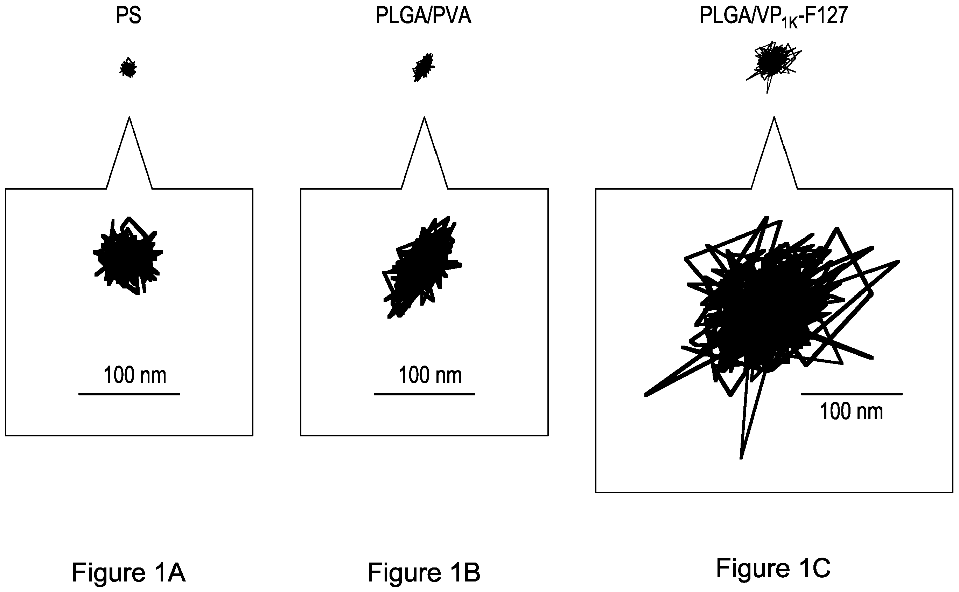

Figures 1A-1E show representative traces of (A) mucoadhesive, uncoated polystyrene particles (PS), (B) poly(lactic acid-co-glycolic acid) PLGA particles coated with polyvinyl-alcohol (PLGA/PVA), (C) PLGA particles coated with poly(ethylene glycol) (PEG) having a molecular weight of about 1000 conjugated to vitamin E (PEG₁₀₀₀-VitE conjugate, or vitamin-E TGPS, or VP1k), followed by coating with Pluronic® F127 (PLGA/VP1k-F127), (D) PLGA particles coated with a PEG having a molecular weight of about 5000 conjugated to vitamin-E (PEG₅₀₀₀-VitE conjugate, or PEG-VitE), followed by coating with Pluronic® F127 (PLGA/VP5k-F127), and (E) polystyrene (PS) particles densely conjugated with 2 kDa PEG (PS-PEG).
Figure 1F shows ensemble-averaged geometric mean square displacements of PLGA/VP5k-F127, PLGA/VP1k-F127, PS-COOH, and PS-PEG particles as a function of time scale.
Figure 1G shows distributions of the logarithms of individual particle effective diffusivities at a time scale of 1 s for PLGA/VP5k-F127 and PLGA/VP1k-F127 particles.
Figure 2A shows an exemplary schematic for the conjugation of methoxy-PEG5k-NH₂ to Vitamin E succinate in the preparation of a PEG-VitE conjugate.
Figures 2B and 2C show ¹³C-NMR spectra of (B) Vitamin E succinate and (C) VP5k.
Figure 3A shows a scanning electron microscope (SEM) image of PLGA particles prepared using Pluronic® F127.
Figure 3B shows an SEM image of PLGA particles prepared using PEG-VitE conjugate.
Figure 3C shows the release of paclitaxel from PLGA/VP5k particles.
Figures 4A-4C show representative trajectories in fresh human cervicovaginal mucus of (A) uncoated PLGA particles, (B) PLGA particles coated with Pluronic® F68, F38, or P65, and (C) particles coated with Pluronic® F127, P103, or P105.
Figure 4D shows a plot of various Pluronics® with different molecular weights of poly(propylene oxide) (PPO) and PEG segments.
Figure 5 shows the correlation between the zeta potential of Pluronic®-coated PLGA particles and the molecular weight of the (A) PPO segment, (B) PEG segment, and (C) entire Pluronic® molecule.
Figure 6A shows ensemble-averaged geometric mean square displacements as a function of time for F127-coated PLGA particles and uncoated PLGA particles in human cervicovaginal mucus.
Figure 6B shows distributions of the logarithms of individual particle effective diffusivities at a time scale of 1 s of the particles given in Figure 6A.
Figure 6C shows the estimated fraction of particles predicted to be capable of penetrating a 30 µm thick mucus layer over time of the particles given in given in Figure 6A.
Figures 7A and 7B show representative trajectories of uncoated particles and particles coated with Pluronic® F127 in CVM.
Figures 7C and 7D show ensemble-averaged geometric mean square displacements as a function of time scale.
Figures 7E and 7F show distributions of the logarithms of individual particle effective diffusivities at a time scale of 1 s.
Figures 7G and 7H show the estimated fraction of particles predicted to be capable of penetrating a 30 µm thick mucus layer over time.
Figures 8A and 8B show trajectories of (A) polystyrene particles administered to fresh human cervicovaginal mucus not treated with Pluronic® (PS_{0%}), and (b) polystyrene particles administered to fresh human cervicovaginal mucus treated with 1% v/v Pluronic® F127 (PS_{1%}).
Figure 8C shows the ensemble-averaged geometric mean square displacements (<MSD>) of PS_{0%}, PS_{1%'} polystyrene particles administered to fresh human cervicovaginal mucus treated with 0.01% v/v Pluronic® F127 (PS_{0.01%}), and polystyrene particles administered to fresh human cervicovaginal mucus treated with 0.0001% v/v Pluronic® F127 (PS_{0.0001%}) as a function of time scale.
Figure 8D shows distributions of the logarithms of effective diffusivities (D_{eff}) at a time scale of 1 s for individual particles of PS_{0%}, PS_{0.0001%}, PS_{0.01%} and PS_{1%}, in mucus treated with Pluronic® F127 as well as polystyrene particles coated with Pluronic® F127 (PS/F127) in native untreated mucus at a time scale of 1 s.
Figure 9 shows a summary of whether polystyrene particles are mobile in fresh human cervicovaginal mucus treated with Pluronic® F68, F38, P65, F127, P103, or P105.

### Detailed Description

The present invention is defined by the claims and generally relates to reducing mucoadhesion of a composition (*e*.*g*., particles). In some cases, particles having reduced mucoadhesion include one or more surface-altering moieties that facilitate passage of the particle through mucus. For example, a particle may be hydrophobic, and the surface-altering moieties may be hydrophilic. The presence of one or more surface-altering moieties may lead to the unexpected property of rapid diffusion through mucus. A particle may be prepared using methods which aid in stabilizing the particles, as described herein. In some cases, a particle described herein may comprise at least one bioactive agent. Additionally, in some cases, pharmaceutical compositions are provided comprising particles described herein and at least one pharmaceutically acceptable excipient. Also methods are provided herein comprising administering to a subject a pharmaceutical composition comprising at least one particle described herein.

Also provided herein is a particle coated with and/or associated with a (poly(ethylene glycol))-(poly(propylene oxide))-(poly(ethylene glycol)) triblock copolymer (hereinafter "PEG-PPO-PEG triblock copolymer"). The molecular weights of the PEG and PPO segments of the PEG-PPO-PEG triblock copolymer may be selected so as to reduce the mucoadhesion of the particle, as described herein. The molecular weight of the PPO block of the PEG-PPO-PEG triblock copolymer may be greater than about 1.8 kDa. In some cases, a particle associated with and/or coated with the triblock copolymer diffuses through mucosal tissues (*e*.*g*., human cervicovaginal mucus) at a diffusivity that is less than 1/500 the diffusivity that the particle diffuses through water.

Without wishing to be bound by theory, a particle coated with and/or associated with a PEG-PPO-PEG triblock copolymer may have reduced mucoadhesion as compared to an uncoated particle due to, at least in part, the display of a plurality of PEG segments on the particle surface. The PPO segment may be adhered to the particle surface (*e*.*g*., in the case of the particle being hydrophobic), thus allowing for a strong association between the particle and the triblock copolymer. In some cases, the PEG-PPO-PEG triblock copolymer is associated with or coating the particle through non-covalent interactions.

The PEG segments of the PEG-PPO-PEG triblock copolymer may function as surface-altering moieties localized on the surface of the particle, and may reduce the adhesion of the particle to mucus. In some cases, the PEG segments of the PEG-PPO-PEG triblock copolymer function as surface-altering moieties which enhance the hydrophilicity of a particle which is otherwise hydrophobic. While not wishing to be bound by theory, one possible mechanism for the reduced mucoadhesion is that PEG alters the microenvironment of the particle, for example, by ordering water and other molecules in the particle/mucus environment. An additional or alternative possible mechanism is that the PEG segments shields the adhesive domains of the mucin fibers, thereby reducing particle adhesion and speeding up particle transport.

The particles described herein may advantageously allow for the coating of a particle with hydrophilic surface-alternating moieties without requiring covalent linking of the surface-altering moieties to the particle surface. This is of particular importance in applications where the particles are to be administered to a mucus surface of a subject. Thus, essentially any known hydrophobic particle (*e*.*g*., comprising a hydrophobic polymeric material) could be associated with and/or coated with a PEG-PPO-PEG triblock copolymer, thereby causing a plurality of surface-altering moieties to be on the particle surface without substantially altering the characteristics of the particle itself. Accordingly, an FDA or otherwise approved particle for administration to a subject (*e*.*g*., a human) could be modified with a triblock copolymer using the techniques and methods described herein and result in reduced mucoadhesion and increased transport of the particles through mucus while the core of the particle remains essentially unaltered.

### Particles with reduced mucoadhesion

The disclosure also may comprise identifying a material such as a particle to which it is desired that its mucoadhesiveness be reduced. Materials in need of increased diffusivity through mucus may, for example, be hydrophobic, have many hydrogen bond donors or acceptors, and/or be highly charged. In some cases, the material may include a hydrophobic polymeric material. The material may then be coated with or associated with a PEG-PPO-PEG triblock copolymer, thereby forming a material with a plurality of surface-altering moieties on the surface, resulting in reduce mucoadhesion. The properties of the particles may be selected based on the desired application and/or properties, as would be understood by one of ordinary skill in the art. Non-liming properties of the particles that may be varied include the size of the particles, the shape of the particles, the composition of the particles, the density of the surface-altering moieties, and the surface charge of the particles, as described herein.

The method may further comprise formulating a pharmaceutical composition of the modified substance, *e*.*g*., in a formulation adapted for delivery (*e*.*g*., topical delivery) to the mucosal surface of a subject. The pharmaceutical composition with surface-altering moieties may be delivered to the mucosal surface of a subject, may pass through the mucosal barrier in the subject, and/or prolonged retention and/or increased uniform distribution of the particles at mucosal surfaces, *e*.*g*., due to reduced mucoadhesion. As will be known by those of ordinary skill in the art, mucus is a viscoelastic and adhesive substance that traps most foreign particles. Trapped particles are not able to reach the underlying epithelium and/or are quickly eliminated by mucus clearance mechanisms. For a particle to reach the underlying epithelium and/or for a particle to have prolonged retention in the mucosal tissue, the particle must quickly penetrate mucus secretions and/or avoid the mucus clearance mechanisms. If a particle does not adhere substantially to the mucosal tissue, the particle may be able to diffuse in the interstitial fluids between mucin fibers and reach the underlying epithelium and/or not be eliminated by the mucus clearance mechanisms. Accordingly, modifying mucoadhesive materials (*e*.*g*., hydrophobic polymeric materials) with a material to reduce the mucoadhesion of the particle may allow for efficient delivery to the particles to the underlying epithelium and/or prolonged retention at mucosal surfaces. A material (*e*.*g*., polymeric particle) associated with and/or coated with a PEG-PPO-PEG triblock copolymer as described herein may pass through a mucosal barrier in a subject, and/or exhibit prolonged retention and/or increase uniform distribution of the particles at mucosal surfaces, *e*.*g*., such substances are cleared more slowly (*e*.*g*., at least 2 times, 5 times, 10 times, or even at least 20 times more slowly) from a subject's body as compared to a particle not associated with and/or not coated with the triblock copolymer.

PEG-PPO-PEG triblock copolymers may be purchased from commercial sources. Such polymers are sold under the trade name Pluronics®. The molecular weight of the PEG blocks and the PPO blocks of the PEG-PPO-PEG triblock copolymers may be selected so as to reduce the mucoadhesion of a particle and to ensure sufficient association of the triblock copolymer with the particle, respectively. As described in the Examples section, the molecular weight of the PPO segment of the PEG-PPO-PEG triblock copolymer may be chosen such that adequate association of the triblock copolymer with the particle occurs, thereby increasing the likelihood that the triblock copolymer remains adhered to the particle. Surprisingly, it has been found that too low of a molecular weight of the PPO segment of the triblock copolymer (*e*.*g*., less than about 1.8 kDa) does not allow for sufficient adhesion between the hydrophobic particle and the triblock copolymer, and thus, the particles with such a triblock copolymer generally do not exhibit sufficient reduced mucoadhesion.

The molecular weight of a PPO block of the PEG-PPO-PEG triblock copolymer may be between about 1.8 kDa and about 10 kDa, or between about 2 kDa and about 10 kDa, or between about 3 kDa and about 10 kDa, or between about 4 kDa and about 10 kDa, or between about 1.8 kDa and about 5 kDa, or between about 3 kDa and about 5 kDa, or between about 2 kDa and about 4 kDa, or between about 2 kDa and about 5 kDa. The molecular weight of the PPO block may be greater than about 1.8 kDa, about 2 kDa, about 3 kDa, about 4 kDa, or about 5 kDa. The molecular weight of the PEG segments may be selected to reduce the mucoadhesion of the particle. In some cases, the molecular weight of a PEG block of the PEG-PPO-PEG triblock copolymers may be greater than about 0.05 kDa, about 0.1 kDa, about 0.2 kDa, about 0.3 kDa, about 0.4 kDa, about 0.5 kDa, about 1 kDa, about 2 kDa, about 3 kDa, about 4 kDa, about 5 kDa, or greater. Pluronics® which may be suitable for use herein include, but are not limited to, F127, F38, F108, F68, F77, F87, F88, F98, L101, L121, L61, L62, L63, L81, L92, P103, P104, P15, P123, P65, P84, and P85. For example, Pluronics® which may be suitable for use herein include, but are not limited to, F127, F108, F77, F87, F88, F98, L101, L121, L61, L62, L63, L81, L92, P103, P104, P15, P123, P84, and P85.

A particle described herein (*e*.*g*., a polymeric particle associated with and/or coated with a PEG-PPO-PEG triblock copolymer) can diffuse through a mucosal barrier at a greater rate or diffusivity than a corresponding particle (*e*.*g*., an unmodified polymeric particle not associated with and/or not coated with a PEG-PPO-PEG triblock copolymer). In some cases, a particle described herein may pass through a mucosal barrier at a rate of diffusivity that is at least 10 times, 20 times, 30 times, 50 times, 100 times, 200 times, 500 times, 1000 times, 2000 times, 5000 times, 10000 times, or more, higher than a corresponding particle. In addition, a particle described herein may pass through a mucosal barrier with a geometric mean squared displacement that is at least 10 times, 20 times, 30 times, 50 times, 100 times, 200 times, 500 times, 1000 times, 2000 times, 5000 times, 10000 times, or more, higher than a corresponding particle. For the purposes of such comparison, the corresponding particle may be approximately the same size, shape, and/or density as the particle described herein lacking the triblock copolymer. In some cases, the measurement is based on a time scale of about 1 second, or about 0.5 second, or about 2 seconds, or about 5 seconds, or about 10 seconds. Those of ordinary skill in the art will be aware of methods for determining the geometric mean square displacement and rate of diffusivity.

A particle described herein may diffuse through a mucosal barrier at a rate approaching the rate or diffusivity at which said particles can diffuse through water. In some cases, a particle described herein may pass through a mucosal barrier at a rate or diffusivity that is at less than 1/100, 1/200, 1/300, 1/400, 1/500, 1/600, 1/700, 1/800, 1/900, 1/1000, 1/2000, 1/5000, 1/10,000 the diffusivity that the particle diffuse through water under identical conditions. A particle described herein may diffuse through human cervicovaginal mucus at a diffusivity that is less than about 1/500 the diffusivity that the particle diffuses through water. In some cases, the measurement is based on a time scale of about 1 second, or about 0.5 second, or about 2 seconds, or about 5 seconds, or about 10 seconds.

Also provided herein are particles that travel through mucus, such as human cervicovaginal mucus, at certain absolute diffusivities. For example, the particles described herein may travel at diffusivities of at least 1 x 10⁻⁴, 2 x 10⁻⁴, 5 x 10⁻⁴, 1 x 10⁻⁴, 2 x 10⁻³, 5 x 10⁻³, 1 x 10⁻², 2 x 10⁻², 4 x 10⁻², 5 x 10⁻², 6 x 10⁻², 8 x 10⁻², 1 x 10⁻¹, 2 x 10⁻¹, 5 x 10⁻¹, 1, or 2 µm/s. In some cases, the measurement is based on a time scale of about 1 second, or about 0.5 second, or about 2 seconds, or about 5 seconds, or about 10 seconds.

A particle described herein may comprise surface-altering moieties at a given density. The surface-altering moieties may be the PEG segments of the PEG-PPO-PEG triblock copolymers. In some cases, the surface-altering moieties are present at a density of between about 0.1 and about 10, or between about 0.1 and about 5, or between about 0.5 and about 5, or between about 0.1 and about 3, or between about 1 and about 10, or between about 0.5 and about 3, or between about 0.9 and about 2.8 surface-altering moieties per nm². In some cases, surface-altering moieties are present at a density of at least 0.001, 0.002, 0.005, 0.01, 0.02, 0.05, 0.1 , 0.2, 0.5, 1 , 2, 5, 10, 20, 50, 100, or more units per nm². Those of ordinary skill in the art will be aware of methods to estimate the average density of surface-altering moieties (see, for example, Wang et al., Angew Chem Int Ed Engl, 2008, 47(50), 9726-9).

Also provided herein are particles comprising surface-altering moieties (*e*.*g*., PEG segments of the PEG-PPO-PEG triblock copolymer) that affect the zeta-potential of the particle, wherein the zeta potential of the coated particle is between -100 mV and 10 mV, between -50 mV and 0 mV, between -40 mV and 0 mV, between -30 mV and 0 mV, between -20 mV and 0 mV, between -10 mV and about 10 mV, between -10 mV and about 0 mV, or between about 0 mV and about 10 mV. In some cases, the zeta potential of a particle described herein is greater than about -30 mV, greater than about -20 mV, greater than about -10 mV, or greater.

In some cases, a particle may be a nanoparticle, *i*.*e*., the particle has a characteristic dimension of less than about 1 micrometer, where the characteristic dimension of a particle is the diameter of a perfect sphere having the same volume as the particle. The plurality of particles may also be characterized by an average diameter (*e*.*g*., the average diameter for the plurality of particles). The diameters of the particles have a Gaussian-type distribution. In some cases, the plurality of particles may have an average diameter greater than about 1 nm, greater than about 5 nm, greater than about 10 nm, greater than about 20 nm, greater than about 50 nm, greater than about 100 nm, greater than about 200 nm, greater than about 300 nm, greater than about 400 nm, greater than about 500 nm, or greater than about 1000 nm in diameter. In some cases, the plurality of the particles have an average diameter of about 1 nm, about 5 nm, about 10 nm, about 25 nm, about 50 nm, about 100 nm, about 150 nm, about 200 nm, about 250 nm, about 300 nm, or about 500 nm *etc.* In some cases, the plurality of particles have an average diameter between about 1 nm and about 1000 nm, between about 50 nm and about 750 nm, between about 100 nm and about 500 nm, or between about 50 nm and about 150 nm.

A particle described herein may comprise a hydrophobic material wherein the hydrophobic material is coated and/or associated with a PEG-PPO-PEG triblock copolymer. The hydrophobic material, in some cases, is a polymeric material and/or a polymeric core. The polymeric material for forming the particle may be any suitable polymer. In some cases, the polymer may be biocompatible and/or biodegradable. In some cases, the polymeric material may comprise more than one type of polymer (*e*.*g*., at least two, three, four, five, or more, polymers). In some cases, a polymer may be a random copolymer or a block copolymer (*e*.*g*., a diblock copolymer, a triblock copolymer).

In some cases, the majority of the particle is formed of a polymeric material. That is, the particle consists of or consists essentially of the polymeric material. In some cases, about 50%, about 60%, about 70%, about 80%, about 90%, about 95%, about 98%, about 99%, or about 100% of the particle is a polymeric material. In some cases, the particle comprises, consists essentially of, or consists of a polymeric material and a bioactive agent. In some cases, a particle described herein may comprise a poly(ethylene glycol)-vitamin E conjugate (hereinafter "PEG-VitE conjugate" or "VP5k"). The PEG-VitE conjugate may be present in the particle due to the technique used for formation of the particle, as described herein.

Non-limiting examples of suitable polymers include polyamines, polyethers, polyamides, polyesters, polycarbamates, polyureas, polycarbonates, polystyrenes, polyimides, polysulfones, polyurethanes, polyacetylenes, polyethylenes, polyethyeneimines, polyisocyanates, polyacrylates, polymethacrylates, polyacrylonitriles, and polyarylates. Non-limiting examples of specific polymers include poly(caprolactone) (PCL), ethylene vinyl acetate polymer (EVA), poly(lactic acid) (PLA), poly(L-lactic acid) (PLLA), poly(glycolic acid) (PGA), poly(lactic acid-co-glycolic acid) (PLGA), poly(L-lactic acid-co-glycolic acid) (PLLGA), poly(D,L-lactide) (PDLA), poly(L- lactide) (PLLA), poly(D,L-lactide-co-caprolactone), poly(D,L-lactide-co-caprolactone-co-glycolide), poly(D,L-lactide-co-PEO-co-D,L-lactide), poly(D,L-lactide-co-PPO-co-D,L-lactide), polyalkyl cyanoacralate, polyurethane, poly-L-lysine (PLL), hydroxypropyl methacrylate (HPMA), polyethyleneglycol, poly-L-glutamic acid, poly(hydroxy acids), polyanhydrides, polyorthoesters, poly(ester amides), polyamides, poly(ester ethers), polycarbonates, polyalkylenes such as polyethylene and polypropylene, polyalkylene glycols such as poly(ethylene glycol) (PEG), polyalkylene oxides (PEO), polyalkylene terephthalates such as poly(ethylene terephthalate), polyvinyl alcohols (PVA), polyvinyl ethers, polyvinyl esters such as poly(vinyl acetate), polyvinyl halides such as poly(vinyl chloride) (PVC), polyvinylpyrrolidone, polysiloxanes, polystyrene (PS), polyurethanes, derivatized celluloses such as alkyl celluloses, hydroxyalkyl celluloses, cellulose ethers, cellulose esters, nitro celluloses, hydroxypropylcellulose, carboxymethylcellulose, polymers of acrylic acids, such as poly(methyl(meth)acrylate) (PMMA), poly(ethyl(meth)acrylate), poly(butyl(meth)acrylate), poly(isobutyl(meth)acrylate), poly(hexyl(meth)acrylate), poly(isodecyl(meth)acrylate), poly(lauryl(meth)acrylate), poly(phenyl(meth)acrylate), poly(methyl acrylate), poly(isopropyl acrylate), poly(isobutyl acrylate), poly(octadecyl acrylate) (jointly referred to herein as "polyacrylic acids"), and copolymers and mixtures thereof, polydioxanone and its copolymers, polyhydroxyalkanoates, polypropylene fumarate), polyoxymethylene, poloxamers, poly(ortho)esters, poly(butyric acid), poly(valeric acid), poly(lactide-co-caprolactone), and trimethylene carbonate, polyvinylpyrrolidone. A polymer may have any suitable molecular weight, wherein the molecular weight is determined using any known technique. Non-limiting examples of techniques include gel permeation chromatography ("GPC"), and light-scattering. Other methods are known in the art.

The polymer may be biocompatible, *i*.*e*., the polymer that does not typically induce an adverse response when inserted or injected into a living subject, for example, it does not include significant inflammation and/or acute rejection of the polymer by the immune system, for instance, via a T-cell-mediated response. It will be recognized, of course, that "biocompatibility" is a relative term, and some degree of immune response is to be expected even for polymers that are highly compatible with living tissue. However, as used herein, "biocompatibility" refers to the acute rejection of material by at least a portion of the immune system, *i*.*e*., a non-biocompatible material implanted into a subject provokes an immune response in the subject that is severe enough such that the rejection of the material by the immune system cannot be adequately controlled, and often is of a degree such that the material must be removed from the subject. One simple test to determine biocompatibility is to expose a polymer to cells *in vitro*; biocompatible polymers are polymers that typically does not result in significant cell death at moderate concentrations, *e*.*g*., at concentrations of about 50 micrograms/10⁶ cells. For instance, a biocompatible polymer may cause less than about 20% cell death when exposed to cells such as fibroblasts or epithelial cells, even if phagocytosed or otherwise uptaken by such cells. Non-limiting examples of biocompatible polymers that may be useful herein include poly(lactic acid-co-glycolic acid) (PLGA), polydioxanone (PDO), polyhydroxyalkanoate, polyhydroxybutyrate, poly(glycerol sebacate), polyglycolide, polylactide, polycaprolactone, or copolymers or derivatives including these and/or other polymers.

A biocompatible polymer may be biodegradable, *i*.*e*., the polymer is able to degrade, chemically and/or biologically, within a physiological environment, such as within the body. For instance, the polymer may be one that hydrolyzes spontaneously upon exposure to water (*e*.*g*., within a subject), and/or the polymer may degrade upon exposure to heat (*e*.*g*., at temperatures of about 37 °C). Degradation of a polymer may occur at varying rates, depending on the polymer or copolymer used. For example, the half-life of the polymer (the time at which 50% of the polymer is degraded into monomers and/or other nonpolymeric moieties) may be on the order of days, weeks, months, or years, depending on the polymer. The polymer may be biologically degraded, *e*.*g*., by enzymatic activity or cellular machinery, in some cases, for example, through exposure to a lysozyme (*e*.*g*., having relatively low pH). In some cases, the polymer may be broken down into monomers and/or other nonpolymeric moieties that cells can either reuse or dispose of without significant toxic effect on the cells (for example, polylactide may be hydrolyzed to form lactic acid, polyglycolide may be hydrolyzed to form glycolic acid, *etc*.). Examples of biodegradable polymers include, but are not limited to, poly(lactide) (or poly(lactic acid)), poly(glycolide) (or poly(glycolic acid)), poly(orthoesters), poly(caprolactones), polylysine, poly(ethylene imine), poly(acrylic acid), poly(urethanes), poly(anhydrides), poly(esters), poly(trimethylene carbonate), poly(ethyleneimine), poly(acrylic acid), poly(urethane), poly(beta amino esters) or the like, and copolymers or derivatives of these and/or other polymers, for example, poly(lactide-co-glycolide) (PLGA).

A polymer may biodegrade within a period that is acceptable in the desired application. For example in *in vivo* therapy, such degradation may occur in a period usually less than about five years, one year, six months, three months, one month, fifteen days, five days, three days, or even one day or less (*e*.*g*., 4-8 hours) on exposure to a physiological solution with a pH between 6 and 8 having a temperature of between 25 and 37 °C. The polymer may degrade in a period of between about one hour and several weeks, depending on the desired application.

In some cases, however, a particle described herein may comprise a hydrophobic material that is not a polymer in addition to a bioactive agent. In some cases, the particle comprises a non-polymeric material which is to be used in connection with mucosal tissue, and wherein reduced mucoadhesion of the particle is required. For example, the particle may comprise a hydrophobic material that strongly associates with mucosal tissue. Coating of the particle with PEG-PPO-PEG triblock copolymer may reduce the mucosal adhesion and allow for better transport of the particle through the mucosal tissue. Non-limiting examples of suitable hydrophobic materials a particle may comprise include certain metals, waxes, and organic materials (*e*.*g*., organic silanes, perfluorinated or fluorinated organic materials).

### Methods for Forming Coated Particles

The particles described herein may be formed using any suitable technique, as will be known to those of ordinary skill in the art. The particles may be formed in the presence of a PEG-PPO-PEG triblock copolymer. Also, the particles may be formed, followed by coating and/or associating the triblock copolymer with the particles. In cases where the particle comprises at least one bioactive agent, the at least one bioactive agent may be encapsulated by and/or adsorbed to the particle material.

Techniques for forming particles will be known to those of ordinary skill in the art and include, for example, (a) phase separation by emulsification and subsequent organic solvent evaporation (including complex emulsion methods such as oil-in-water emulsions, water-in-oil emulsions, and water-oil-water emulsions); (b) coacervation-phase separation; (c) melt dispersion; (d) interfacial deposition; (e) in situ polymerization; (f) spray-drying and spray-congealing; (g) air suspension coating; (h) pan and spray coating; (i) freeze-drying, air drying, vacuum drying, fluidized-bed drying; precipitation (*e*.*g*., nanoprecipitation, microprecipitation); and (j) critical fluid extraction. The shape of the particles may be determined by scanning or transmission electron microscopy, or other techniques known to those of ordinary skill in the art. Spherically shaped particles may be generally used, *e*.*g*., for circulation through the bloodstream. If desired, the particles may be fabricated using known techniques into other shapes that are more useful for a specific application.

The particles may first be formed using precipitation techniques, following by coating of the particles with a triblock copolymer. Precipitation techniques (*e*.*g*., microprecipitation techniques, nanoprecipitation techniques) may involve forming a first solution comprising the polymeric material (or other hydrophobic material) and a solvent, wherein the polymeric material is substantially soluble in the solvent. The solution may be added to a second solution comprising another solvent in which the polymeric material is substantially insoluble, thereby forming a plurality of particles comprising the polymeric material. In some cases, one or more surfactants, materials, and/or bioactive agents may be present in the first and/or second solution.

In an exemple, a method of forming the particles includes using a poly(ethylene glycol)-vitamin E conjugate (hereinafter "PEG-VitE conjugate" or "VP5k"). The PEG-VitE conjugate can act as a surfactant, may aid in stabilizing the particles, and/or may aid in encapsulating the particle material. In some cases, a method for forming a plurality of particles using PEG-VitE comprises forming a solution comprising a polymeric material (or other hydrophobic material), and adding the solution to a solvent in which the polymeric material is substantially insoluble. The PEG-VitE conjugate may be present in the solution comprising the polymeric material and/or the solvent to which the solution is present. Upon addition of the solution comprising the polymeric material to the solvent, a plurality of particles form, which are stabilized by the PEG-VitE conjugate. The PEG-VitE conjugate may be present in the solvent or solution at about 0.1%, 0.5%, 1.0%, 1.5%, 1.65%, 2%, 3%, 4%, 5%, 10%, 20% weight percent, or greater. Examples of solvents that may be suitable for use herein include, but are not limited to, acetonitrile, benzene, *p*-cresol, toluene, xylene, mesitylene, diethyl ether, glycol, petroleum ether, hexane, cyclohexane, pentane, dichloromethane (methylene chloride), chloroform, carbon tetrachloride, dioxane, tetrahydrofuran (THF), dimethyl sulfoxide, dimethylformamide, hexamethyl-phosphoric triamide, ethyl acetate, pyridine, triethylamine, picoline, mixtures thereof, or the like.

Following formation of the plurality of particles, the particles may be exposed to a solution comprising a PEG-PPO-PEG triblock copolymer, and the triblock copolymer may associate with and/or coat the particles, thereby forming particles described herein. For example, the particles may be washed with a solution comprising the triblock copolymer. The solution comprising the triblock copolymer may comprise about 0.1%, 0.5%, 1.0%, 1.5%, 1.65%, 2%, 3%, 4%, 5%, 10%, 20% weight percent, or more, of the triblock copolymer.

The particles associated with and/or coated with the triblock copolymer may or may not comprise PEG-VitE conjugate. In some cases, the PEG-VitE conjugate may be substantially replaced and/or displaced by the triblock copolymer. In other cases, at least some of the PEG-VitE conjugate remains associated with the particle, and the PEG portion of the PEG-VitE conjugate may function as a surface-altering moiety.

As a specific example of a method for forming a plurality of coated particles, a solution may be prepared comprising the polymeric material and an organic solvent, wherein the polymeric material is substantially soluble in the organic solvent (*e*.*g*., the polymeric materials may be PLGA and PCL, and the solvent may be tetrahydrofuran). The solution may be added dropwise to a copious amount of aqueous solution (*e*.*g*., at least about 10 times, at least about 20 times, at least about 30 times, at least about 40 times, at least about 50 times, or greater, the amount of organic solvent by volume), thereby causing a plurality of particles to form. The organic solvent may be removed (*e*.*g*., by evaporation, heating, *etc*.) and the particles may be isolated using techniques known to those of ordinary skill in the art (*e*.*g*., centrifugation, filtering, *etc*.). The particles may then be washed with a solution comprising the triblock copolymer (*e*.*g*., an aqueous solution comprising a PEG-PPO-PEG triblock copolymer), thereby forming a plurality of particles coated with and/or associated with the triblock copolymer. The coated particles may or may not be purified, for example, to remove any aggregated particles. At least one bioactive agent may be present in solution which contained a solvent and the polymeric material, and the resulting particle may additionally comprise the bioactive agent. The bioactive agent may also be incorporated into the particles using other methods or techniques, as will be known to one of ordinary skill in the art.

As another specific method, a particle may be associated with or coated with a triblock copolymer by incubating (*e*.*g*., in solution) the particle with the triblock copolymer for a period of about 1 minutes, about 2 minutes, about 5 minutes, about 10 minutes, about 15 minutes, about 20 minutes, about 30 minutes, about 60 minutes, or more.

In some cases, the molecular weight of the poly(ethylene glycol) of the PEG-VitE conjugate is greater than about 2 kDa. The molecular weight of the poly(ethylene glycol) of the PEG-VitE conjugate may be selected so as to aid in the formation and/or transport of the particle across a mucosal barrier of the particles. Use of a PEG-VitE conjugate with a poly(ethylene glycol) having a molecular weight greater than about 2 kDa may allow for greater penetration of the particles through a mucosal barrier as compared to use of a PEG-VitE conjugate with a poly(ethylene glycol) having a molecular weight less than about 2 kDa. The higher molecular weight poly(ethylene glycol) may allow for mucus-penetration performance that is not observed with poly(ethylene glycol) having a molecular weight less than about 2 kDa. Additionally, the higher molecular weight poly(ethylene glycol) may facilitate drug encapsulation as compared to other commonly used surfactants. The combined ability to act as a surfactant and to reduce mucoadhesion provides important benefits as compared to other commonly used surfactants for drug encapsulation. In some cases, the molecular weight of the poly(ethylene glycol) of the PEG-VitE conjugate is between about 2 kDa and about 8 kDa, or between about 3 kDa and about 7 kDa, or between about 4 kDa and about 6 kDa, or between about 4.5 kDa and about 6.5 kDa, or about 5 kDa.

PEG-VitE conjugates may be synthesized using techniques known to those of ordinary skill in the art. A non-limiting example of the synthesis of a PEG-VitE conjugate, wherein the poly(ethylene glycol) portion of the conjugate has a molecular weight of about 5 kDa is described in Example 1.

It should be noted, that the vitamin-E portion of the PEG-VitE conjugate may be substituted with other suitable components. For example, the vitamin E may be substituted with another vitamin (*e*.*g*., vitamin A), cholesterol, *etc.* In some cases, the vitamin-E portion of the PEG-VitE conjugate may be substituted with a hydrophobic moiety. In some cases, the vitamin-E portion of the PEG-VitE conjugate may be substituted with the hydrophobic component of other surfactants, *e*.*g*., an ionic or non-ionic surfactant. Non-limiting examples of non-ionic surfactants include polysorbates such as those comprising cholates, monolaurates, monooleates; Polysorbate 80 (*e*.*g*., TWEEN 80®), Polysorbate 20, (*e*.*g*., TWEEN 20®), polyoxyethylene alkyl ethers (*e*.*g*. Brij 35®, and Brij 58®), as well as others, including Triton X-100®, Triton X-114®, NP-40®, Span 85. Non-limiting examples of hydrophobic components of a surfactant include sterol chains, fatty acids, hydrocarbon chains (including fluorocarbonated chains), and alkylene oxide chains.

### Uses

The particles described herein may be employed in any suitable application. In some cases, the particles are part of a pharmaceutical compositions (*e*.*g*., as described herein), for example, those used to deliver a bioactive agent through or to a mucosal surface. A pharmaceutical composition may comprise at least one particle described herein and one or more pharmaceutically acceptable excipients. The composition may be used in treating, preventing, and/or diagnosing a condition in a subject, wherein the method comprises administering to a subject the pharmaceutical composition.

A pharmaceutical composition of the described herein may be delivered to a mucosal surface in a subject and may pass through a mucosal barrier in the subject, and/or may exhibit prolonged retention and/or increased uniform distribution of the particles at mucosal surfaces, *e*.*g*., due to reduced mucoadhesion. Non-limiting examples of mucosal tissues include oral (*e*.*g*., including the buccal and esophagal membranes and tonsil surface), ophthalmic, gastrointestinal (*e*.*g*., including stomach, small intestine, large intestine, colon, rectum), nasal, respiratory (*e*.*g*., including nasal, pharyngeal, tracheal and bronchial membranes), genital (*e*.*g*., including vaginal, cervical and urethral membranes).

Pharmaceutical compositions containing the particles described herein may be administered to a subject via any route known in the art. These include, but are not limited to, oral, sublingual, nasal, intradermal, subcutaneous, intramuscular, rectal, vaginal, intravenous, intraarterial, and inhalational administration. As would be appreciated by one of skill in this art, the route of administration and the effective dosage to achieve the desired biological effect is determined by the agent being administered, the target organ, the preparation being administered, time course of administration, disease being treated, *etc.* As an example, the particles may be included in a pharmaceutical composition to be formulated as a nasal spray, such that the pharmaceutical composition is delivered across a nasal mucus layer. As another example, the particles may be included in a pharmaceutical composition to be formulated as an inhaler, such that the pharmaceutical compositions is delivered across a pulmonary mucus layer. Similarly, the particles may be included in a pharmaceutical composition that is to be delivered via oral, ophthalmic, gastrointestinal, nasal, respiratory, rectal, urethral and/or vaginal tissues.

### Administration of a (Poly(ethylene glycol))-(poly(propylene oxide))-(poly(ethylene glycol)) Triblock Copolymer and Particles to Mucosal Tissues

Also provided herein is administration of at least one particle and a (poly(ethylene glycol))-(poly(propylene oxide))-(poly(ethylene glycol)) triblock copolymer to a subject. That is, "free" (poly(ethylene glycol))-(poly(propylene oxide))-(poly(ethylene glycol)) triblock copolymer may be administered to a subject, wherein the (poly(ethylene glycol))-(poly(propylene oxide))-(poly(ethylene glycol)) triblock copolymer is not associated with the particles prior to administration of the particle and/or triblock copolymer to the subject. The (poly(ethylene glycol))-(poly(propylene oxide))-(poly(ethylene glycol)) triblock copolymer may be administered to a subject prior to, during, and/or following administration of the particles to the subject. The (poly(ethylene glycol))-(poly(propylene oxide))-(poly(ethylene glycol)) triblock copolymer may or may not associate with a particle following administration of both the triblock copolymer and the particles to a subject.

In some cases, the administration of a (poly(ethylene glycol))-(poly(propylene oxide))-(poly(ethylene glycol)) triblock copolymer to the subject prior to, during, and/or following the administration of particles may increase the rate of transport of the particles through the mucus as compared to the mean square displacement of the particles in the absence of the administration of the (poly(ethylene glycol))-(poly(propylene oxide))-(poly(ethylene glycol)) triblock copolymer, under essentially identical conditions. Without wishing to be bound by any particular theory, the administration of the (poly(ethylene glycol))-(poly(propylene oxide))-(poly(ethylene glycol)) triblock copolymer may increase the mean square displacement of a plurality of particles by associating with the particles and/or the mucus, thereby reducing the adhesion of the particles with mucus mesh. For example, in some cases, the (poly(ethylene glycol))-(poly(propylene oxide))-(poly(ethylene glycol)) triblock copolymer may increase particle transport in mucus by masking hydrophobic domains along mucin fibers that may trap mucoadhesive particles, instead of coating the particles surface. In some cases, the mean square displacement is increased 1.1 times, 1.25 times, 1.5 times, 1.75 times, 2.0 times, 3 times, 4 times, 5 times, 10 times, 15 times. 20 times, 30 times, 40 times, 50 times, 75 times, 100 times, or more, as compared to the mean square displacement of the particles administered in the absence of the (poly(ethylene glycol))-(poly(propylene oxide))-(poly(ethylene glycol)) triblock copolymer.

As mentioned herein, the (poly(ethylene glycol))-(poly(propylene oxide))-(poly(ethylene glycol)) triblock copolymer may be administered to a subject prior to, during, and/or following administration of the particles to the subject. For example, in some cases, the (poly(ethylene glycol))-(poly(propylene oxide))-(poly(ethylene glycol)) triblock copolymer may be administered 1 second, 2 seconds, 3 seconds, 5 seconds, 10 seconds, 20 seconds, 30 seconds, 1 minute, 2 minutes, 5 minutes, 10 minutes, 30 minutes, or more, prior to or following administration of the plurality of particles. The triblock copolymer may be administered in one dose, or more than one dose (*e*.*g*., two doses, three doses, four doses, etc.). In cases where more than one dose is administered to a subject, the doses may be administered to the subject at different locations and/or at different time points (*e*.*g*., one dose prior to administration of the particles and one dose following administration of the particles).

The (poly(ethylene glycol))-(poly(propylene oxide))-(poly(ethylene glycol)) triblock copolymer may be provided at 0.05% w/v, 0.1% w/v, 0.02% w/v, 0.3% w/v, 0.4% w/v, 0.5% w/v, 0.6% w/v, 0.7% w/v, 0.8% w/v, 0.9% w/v, 1.0% w/v, 1.5% w/v, 2.0% w/v, 3.0% w/v, 4.0% w/v, 5.0% w/v, 10% w/v, 20% w/v, 30% w/v, 40% w/v, 50% w/v, 60% w/v, 70% w/v, 80% w/v, 90% w/v, 100% w/v, or more, of the triblock copolymer in a liquid (*e*.*g*., water, buffer, *etc*.). In some cases, the copolymer may be provided as an aqueous solution. The amount of (poly(ethylene glycol))-(poly(propylene oxide))-(poly(ethylene glycol)) triblock copolymer administered to a subject may be about0.1 % w/v, 0.05% w/v, 0.1% w/v, 0.02% w/v, 0.3% w/v, 0.4% w/v, 0.5% w/v, 0.6% w/v, 0.7% w/v, 0.8% w/v, 0.9% w/v, 1.0% w/v, 1.5% w/v, 2.0% w/v, 3.0% w/v, 4.0% w/v, 5.0% w/v, 10% w/v, 20% w/v, 30% w/v, 40% w/v, 50% w/v, 60% w/v, 70% w/v, 80% w/v, 90% w/v, 100% w/v, or more, of weight of copolymer per volume of mucus. The ratio of (poly(ethylene glycol))-(poly(propylene oxide))-(poly(ethylene glycol)) triblock copolymer administered to the particles administered may be about 50:1, 40:1, 30:1, 20:1, 15:1, 10:1, 5:1,4:1,3:1,2:1, 1:1, 1:2, 1:3, 1:4, 1:5, 1:6, 1:10, 1:15, 1:20, 1:30, 1:40, or 1:50, by volume.. The ratio of (poly(ethylene glycol))-(poly(propylene oxide))-(poly(ethylene glycol)) triblock copolymer administered to the particles administered may be about 1000:1, 5000:1, 250:1, 100:1, 50:1, 40:1, 30:1, 20:1, 15:1, 10:1, 5:1, 4:1, 3:1, 2:1, 1:1, 1:2, 1:3, 1:4, 1:5, 1:6, 1:10, 1:15, 1:20, 1:30, 1:40, 1:50, 1:100, 1:250, 1:500, or 1:1000, by weight%.

The particles and the (poly(ethylene glycol))-(poly(propylene oxide))-(poly(ethylene glycol)) triblock copolymer may be as described herein. In some cases, the particles chosen for use with this case may be selected because the transport of the particles is slowed in mucus (*e*.*g*., due to hydrophobic interactions). In some cases, the particles comprise a bioactive agent (*e*.*g*., one or more bioactive agents). The particles may comprise a polymeric material (*e*.*g*., as described herein). The molecular weight of the (poly(propylene oxide)) block of the (poly(ethylene glycol))-(poly(propylene oxide))-(poly(ethylene glycol)) triblock copolymer may be greater than about 1.8 kDa.

### Bioactive Agents

A coated particle may comprise at least one bioactive agent (*e*.*g*., a drug or medicament). The bioactive agent may be encapsulated in the particle and/or may be disposed on the surface of the particle. In some cases, the bioactive agent may be encapsulated in the particle (or particle core) prior to or following coating and/or association of the particle with a PEG-PPO-PEG triblock copolymer. The bioactive agent may be may be disposed on the surface of a particle and/or contained within a particle using commonly known techniques (*e*.*g*., by coating, adsorption, covalent linkage, or other process). In some cases, the bioactive agent is present during the formation of the particle, as described herein.

Non-limiting examples of bioactive agents include imaging agents, diagnostic agents, therapeutic agents, agents with a detectable label, nucleic acids, nucleic acid analogs, small molecules, peptidomimetics, proteins, peptides, lipids, or surfactants.

A number of drugs that are mucoadhesive are known in the art (see, for example, Khanvilkar K, Donovan MD, Flanagan DR, Drug transfer through mucus, Advanced Drug Delivery Reviews 48 (2001) 173-193; Bhat PG, Flanagan DR, Donovan MD. Drug diffusion through cystic fibrotic mucus: steady-state permeation, rheologic properties, and glycoprotein morphology, J Pharm Sci, 1996 Jun;85(6):624-30.). Additional non-limiting examples of bioactive agents include imaging and diagnostic agents (such as radioopaque agents, labeled antibodies, labeled nucleic acid probes, dyes, such as colored or fluorescent dyes, *etc*.) and adjuvants (radiosensitizers, transfection-enhancing agents, chemotactic agents and chemoattractants, peptides that modulate cell adhesion and/or cell mobility, cell permeabilizing agents, vaccine potentiators, inhibitors of multidrug resistance and/or efflux pumps, *etc*.). The bioactive agent may be paclitaxel. Additional non-limiting examples of bioactive agents include aloxiprin, auranofin, azapropazone, benorylate, diflunisal, etodolac, fenbufen, fenoprofen calcim, flurbiprofen, ibuprofen, indomethacin, ketoprofen, meclofenamic acid, mefenamic acid, nabumetone, naproxen, oxyphenbutazone, phenylbutazone, piroxicam, sulindac, albendazole, bephenium hydroxynaphthoate, cambendazole, dichlorophen, ivermectin, mebendazole, oxamniquine, oxfendazole, oxantel embonate, praziquantel, pyrantel embonate, thiabendazole, amiodarone HCl, disopyramide, flecainide acetate, quinidine sulphate. Anti-bacterial agents: benethamine penicillin, cinoxacin, ciprofloxacin HCl, clarithromycin, clofazimine, cloxacillin, demeclocycline, doxycycline, erythromycin, ethionamide, imipenem, nalidixic acid, nitrofurantoin, rifampicin, spiramycin, sulphabenzamide, sulphadoxine, sulphamerazine, sulphacetamide, sulphadiazine, sulphafurazole, sulphamethoxazole, sulphapyridine, tetracycline, trimethoprim, dicoumarol, dipyridamole, nicoumalone, phenindione, amoxapine, maprotiline HCl, mianserin HCL, nortriptyline HCl, trazodone HCL, trimipramine maleate, acetohexamide, chlorpropamide, glibenclamide, gliclazide, glipizide, tolazamide, tolbutamide, beclamide, carbamazepine, clonazepam, ethotoin, methoin, methsuximide, methylphenobarbitone, oxcarbazepine, paramethadione, phenacemide, phenobarbitone, phenytoin, phensuximide, primidone, sulthiame, valproic acid, amphotericin, butoconazole nitrate, clotrimazole, econazole nitrate, fluconazole, flucytosine, griseofulvin, itraconazole, ketoconazole, miconazole, natamycin, nystatin, sulconazole nitrate, terbinafine HCl, terconazole, tioconazole, undecenoic acid, allopurinol, probenecid, sulphin-pyrazone, amlodipine, benidipine, darodipine, dilitazem HCl, diazoxide, felodipine, guanabenz acetate, isradipine, minoxidil, nicardipine HCl, nifedipine, nimodipine, phenoxybenzamine HCl, prazosin HCL, reserpine, terazosin HCL, amodiaquine, chloroquine, chlorproguanil HCl, halofantrine HCl, mefloquine HCl, proguanil HCl, pyrimethamine, quinine sulphate, dihydroergotamine mesylate, ergotamine tartrate, methysergide maleate, pizotifen maleate, sumatriptan succinate, atropine, benzhexol HCl, biperiden, ethopropazine HCl, hyoscyamine, mepenzolate bromide, oxyphencylcimine HCl, tropicamide, aminoglutethimide, amsacrine, azathioprine, busulphan, chlorambucil, cyclosporin, dacarbazine, estramustine, etoposide, lomustine, melphalan, mercaptopurine, methotrexate, mitomycin, mitotane, mitozantrone, procarbazine HCl, tamoxifen citrate, testolactone, benznidazole, clioquinol, decoquinate, diiodohydroxyquinoline, diloxanide furoate, dinitolmide, furzolidone, metronidazole, nimorazole, nitrofurazone, ornidazole, tinidazole, carbimazole, propylthiouracil, alprazolam, amylobarbitone, barbitone, bentazepam, bromazepam, bromperidol, brotizolam, butobarbitone, carbromal, chlordiazepoxide, chlormethiazole, chlorpromazine, clobazam, clotiazepam, clozapine, diazepam, droperidol, ethinamate, flunanisone, flunitrazepam, fluopromazine, flupenthixol decanoate, fluphenazine decanoate, flurazepam, haloperidol, lorazepam, lormetazepam, medazepam, meprobamate, methaqualone, midazolam, nitrazepam, oxazepam, pentobarbitone, perphenazine pimozide, prochlorperazine, sulpiride, temazepam, thioridazine, triazolam, zopiclone, acebutolol, alprenolol, atenolol, labetalol, metoprolol, nadolol, oxprenolol, pindolol, propranolol, amrinone, digitoxin, digoxin, enoximone, lanatoside C, medigoxin, beclomethasone, betamethasone, budesonide, cortisone acetate, desoxymethasone, dexamethasone, fludrocortisone acetate, flunisolide, flucortolone, fluticasone propionate, hydrocortisone, methylprednisolone, prednisolone, prednisone, triamcinolone, acetazolamide, amiloride, bendrofluazide, bumetanide, chlorothiazide, chlorthalidone, ethacrynic acid, frusemide, metolazone, spironolactone, triamterene, bromocriptine mesylate, lysuride maleate, bisacodyl, cimetidine, cisapride, diphenoxylate HCl, domperidone, famotidine, loperamide, mesalazine, nizatidine, omeprazole, ondansetron HCL, ranitidine HCl, sulphasalazine, acrivastine, astemizole, cinnarizine, cyclizine, cyproheptadine HCl, dimenhydrinate, flunarizine HCl, loratadine, meclozine HCl, oxatomide, terfenadine, bezafibrate, clofibrate, fenofibrate, gemfibrozil, probucol, amyl nitrate, glyceryl trinitrate, isosorbide dinitrate, isosorbide mononitrate, pentaerythritol tetranitrate, betacarotene, vitamin A, vitamin B 2 , vitamin D, vitamin E, vitamin K, codeine, dextropropyoxyphene, diamorphine, dihydrocodeine, meptazinol, methadone, morphine, nalbuphine, pentazocine, clomiphene citrate, danazol, ethinyl estradiol, medroxyprogesterone acetate, mestranol, methyltestosterone, norethisterone, norgestrel, estradiol, conjugated oestrogens, progesterone, stanozolol, stibestrol, testosterone, tibolone, amphetamine, dexamphetamine, dexfenfluramine, fenfluramine, and mazindol.

The particles described herein comprising a bioactive agent may be administered to a subject to be delivered in an amount sufficient to deliver to a subject a therapeutically effective amount of an incorporated bioactive agent as part of a diagnostic, prophylactic, or therapeutic treatment. The desired concentration of bioactive agent in the particle will depend on numerous factors, including, but not limited to, absorption, inactivation, and excretion rates of the drug as well as the delivery rate of the compound from the subject compositions. It is to be noted that dosage values may also vary with the severity of the condition to be alleviated. It is to be further understood that for any particular subject, specific dosage regimens should be adjusted over time according to the individual need and the professional judgment of the person administering or supervising the administration of the compositions. Typically, dosing will be determined using techniques known to one skilled in the art.

The concentration and/or amount of any bioactive agent to be administered to a subject may be readily determined by one of ordinary skill in the art. Known methods are also available to assay local tissue concentrations, diffusion rates from particles and local blood flow before and after administration of therapeutic formulations according to the disclosure.

A particle described herein may further comprise a targeting agent or molecule to aid in directing the particle to a specific tissue or location in the subject's body. The targeting moiety may be attached to the particle or to one or more of the surface-altering moieties of the coated particle using methods known to those of ordinary skill in the art.

### Pharmaceutical Composition

Once the particles have been prepared, they may be combined with one or more pharmaceutically acceptable excipients to form a pharmaceutical composition that is suitable to administer to subjects, including humans. As would be appreciated by one of skill in this art, the excipients may be chosen based on the route of administration as described below, the agent being delivered, time course of delivery of the agent, *etc.*

Pharmaceutical compositions described herein and for use in accordance with the present disclosure may include a pharmaceutically acceptable excipient or carrier. As used herein, the term "pharmaceutically acceptable excipient" or "pharmaceutically acceptable carrier" means a non-toxic, inert solid, semi-solid or liquid filler, diluent, encapsulating material or formulation auxiliary of any type. Some examples of materials which can serve as pharmaceutically acceptable carriers are sugars such as lactose, glucose, and sucrose; starches such as corn starch and potato starch; cellulose and its derivatives such as sodium carboxymethyl cellulose, ethyl cellulose, and cellulose acetate; powdered tragacanth; malt; gelatin; talc; excipients such as cocoa butter and suppository waxes; oils such as peanut oil, cottonseed oil; safflower oil; sesame oil; olive oil; corn oil and soybean oil; glycols such as propylene glycol; esters such as ethyl oleate and ethyl laurate; agar; detergents such as Tween 80; buffering agents such as magnesium hydroxide and aluminum hydroxide; alginic acid; pyrogen-free water; isotonic saline; Ringer's solution; ethyl alcohol; and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator.

The pharmaceutical compositions described herein can be administered to humans and/or to animals, orally, rectally, parenterally, intracisternally, intravaginally, intranasally, intraperitoneally, topically (as by powders, creams, ointments, or drops), bucally, or as an oral or nasal spray. The mode of administration will vary depending on the intended use, as is well known in the art. For example, if compositions are to be administered orally, it may be formulated as tablets, capsules, granules, powders, or syrups. Alternatively, formulations described herein may be administered parenterally as injections (intravenous, intramuscular, or subcutaneous), drop infusion preparations, or suppositories. For application by the ophthalmic mucous membrane route, subject compositions may be formulated as eyedrops or eye ointments. These formulations may be prepared by conventional means, and, if desired, the subject compositions may be mixed with any conventional additive, such as a binder, a disintegrating agent, a lubricant, a corrigent, a solubilizing agent, a suspension aid, an emulsifying agent, or a coating agent. In addition, subject compositions described herein may be lyophilized or subjected to another appropriate drying technique such as spray drying.

Particles described herein may be administered in inhalant or aerosol formulations described herein may comprise one or more bioactive agents, such as adjuvants, diagnostic agents, imaging agents, or therapeutic agents useful in inhalation therapy. The particle size of the particulate medicament should be such as to permit inhalation of substantially all of the medicament into the lungs upon administration of the aerosol formulation and will thus desirably be less than 20 microns, preferably in the range 1 to 10 microns, *e*.*g*., 1 to 5 microns. The particle size of the medicament may be reduced by conventional means, for example by milling or micronisation. The final aerosol formulation may contain between 0.005-90% w/w, or between 0.005-50%, or between about 0.005-5% w/w, or between 0.01-1.0% w/w, of medicament relative to the total weight of the formulation.

It is desirable, but by no means required, that the formulations described herein contain no components which may provoke the degradation of stratospheric ozone. In particular, propellants are selected that do not contain or do not consist essentially of chlorofluorocarbons such as CCl₃F, CCl₂F₂, and CF₃CCl₃.

The aerosol may comprise propellant. The propellant may optionally contain an adjuvant having a higher polarity and/or a higher boiling point than the propellant. Polar adjuvants which may be used include (*e*.*g*., C₂₋₆) aliphatic alcohols and polyols such as ethanol, isopropanol, and propylene glycol, preferably ethanol. In general, only small quantities of polar adjuvants (*e*.*g*., 0.05-3.0% w/w) may be required to improve the stability of the dispersion-the use of quantities in excess of 5% w/w may tend to dissolve the medicament. Formulations described herein may contain less than 1% w/w, *e*.*g*., about 0.1% w/w, of polar adjuvant. However, the formulations described herein may be substantially free of polar adjuvants, especially ethanol. Suitable volatile adjuvants include saturated hydrocarbons such as propane, n-butane, isobutane, pentane and isopentane and alkyl ethers such as dimethyl ether. In general, up to 50% w/w of the propellant may comprise a volatile adjuvant, for example 1 to 30% w/w of a volatile saturated C₁-C₆ hydrocarbon. Optionally, the aerosol formulations described herein may further comprise one or more surfactants. The surfactants can be physiologically acceptable upon administration by inhalation. Within this category are included surfactants such as L-α-phosphatidylcholine (PC), 1,2-dipalmitoylphosphatidycholine (DPPC), oleic acid, sorbitan trioleate, sorbitan mono-oleate, sorbitan monolaurate, polyoxyethylene (20) sorbitan monolaurate, polyoxyethylene (20) sorbitan monooleate, natural lecithin, oleyl polyoxyethylene (2) ether, stearyl polyoxyethylene (2) ether, lauryl polyoxyethylene (4) ether, block copolymers of oxyethylene and oxypropylene, synthetic lecithin, diethylene glycol dioleate, tetrahydrofurfuryl oleate, ethyl oleate, isopropyl myristate, glyceryl monooleate, glyceryl monostearate, glyceryl monoricinoleate, cetyl alcohol, stearyl alcohol, polyethylene glycol 400, cetyl pyridinium chloride, benzalkonium chloride, olive oil, glyceryl monolaurate, corn oil, cotton seed oil, and sunflower seed oil. Preferred surfactants are lecithin, oleic acid, and sorbitan trioleate.

The formulations described herein may be prepared by dispersal of the particles in the selected propellant and/or co-propellant in an appropriate container, *e*.*g*., with the aid of sonication. The particles may be suspended in co-propellant and filled into a suitable container. The valve of the container is then sealed into place and the propellant introduced by pressure filling through the valve in the conventional manner. The particles may be thus suspended or dissolved in a liquified propellant, sealed in a container with a metering valve and fitted into an actuator. Such metered dose inhalers are well known in the art. The metering valve may meter 10 to 500 µL and preferably 25 to 150 µL. Dispersal may be achieved using dry powder inhalers (*e*.*g*., spinhaler) for the particles (which remain as dry powders). Also, nanospheres, may be suspended in an aqueous fluid and nebulized into fine droplets to be aerosolized into the lungs.

Sonic nebulizers may be used because they minimize exposing the agent to shear, which may result in degradation of the particles. Ordinarily, an aqueous aerosol is made by formulating an aqueous solution or suspension of the particles together with conventional pharmaceutically acceptable carriers and stabilizers. The carriers and stabilizers vary with the requirements of the particular composition, but typically include non-ionic surfactants (Tweens, Pluronic®, or polyethylene glycol), innocuous proteins like serum albumin, sorbitan esters, oleic acid, lecithin, amino acids such as glycine, buffers, salts, sugars, or sugar alcohols. Aerosols generally are prepared from isotonic solutions. Ophthalmic formulations, eye ointments, powders, solutions and the like, are also contemplated herein.

Liquid dosage forms for oral administration include pharmaceutically acceptable emulsions, microemulsions, solutions, suspensions, syrups, and elixirs. In addition to the active ingredients (*i*.*e*., microparticles, nanoparticles, liposomes, micelles, polynucleotide/lipid complexes), the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension, or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables. The particles may be suspended in a carrier fluid comprising 1% (w/v) sodium carboxymethyl cellulose and 0.1% (v/v) Tween 80.

The injectable formulations can be sterilized, for example, by filtration through a bacteria-retaining filter, or by incorporating sterilizing agents in the form of sterile solid compositions which can be dissolved or dispersed in sterile water or other sterile injectable medium prior to use.

Compositions for rectal or vaginal administration can be suppositories which can be prepared by mixing the particles with suitable non-irritating excipients or carriers such as cocoa butter, polyethylene glycol, or a suppository wax which are solid at ambient temperature but liquid at body temperature and therefore melt in the rectum or vaginal cavity and release the particles.

Solid dosage forms for oral administration include capsules, tablets, pills, powders, and granules. In such solid dosage forms, the particles are mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets, and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like.

The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

Solid compositions of a similar type may also be employed as fillers in soft and hard-filled gelatin capsules using such excipients as lactose or milk sugar as well as high molecular weight polyethylene glycols and the like.

Dosage forms for topical or transdermal administration of an inventive pharmaceutical composition include ointments, pastes, creams, lotions, gels, powders, solutions, sprays, inhalants, or patches. The particles are admixed under sterile conditions with a pharmaceutically acceptable carrier and any needed preservatives or buffers as may be required. Ophthalmic formulation, ear drops, and eye drops are also contemplated herein.

The ointments, pastes, creams, and gels may contain, in addition to the particles described herein, excipients such as animal and vegetable fats, oils, waxes, paraffins, starch, tragacanth, cellulose derivatives, polyethylene glycols, silicones, bentonites, silicic acid, talc, and zinc oxide, or mixtures thereof.

Powders and sprays can contain, in addition to the particles described herein, excipients such as lactose, talc, silicic acid, aluminum hydroxide, calcium silicates, and polyamide powder, or mixtures of these substances. Sprays can additionally contain customary propellants such as chlorofluorohydrocarbons.

Transdermal patches have the added advantage of providing controlled delivery of a compound to the body. Such dosage forms can be made by dissolving or dispensing the microparticles or nanoparticles in a proper medium. Absorption enhancers can also be used to increase the flux of the compound across the skin. The rate can be controlled by either providing a rate controlling membrane or by dispersing the particles in a polymer matrix or gel.

### Definitions

"Hydrophobic" and "hydrophilic" are given their ordinary meaning in the art and, as will be understood by those skilled in the art, in many instances herein, these are relative terms. With respect to a substantially hydrophilic drug or drug precursor, this means a molecule that has appreciable solubility in an aqueous environment. In some cases, the hydrophilic drug may be substantially soluble in water (*e*.*g*., at least about 1 g/L, at least about 5 g/L, at least about 10 g/L, *etc*.).

The term "biocompatible," as used herein is intended to describe compounds that are not toxic to cells. Compounds are "biocompatible" if their addition to cells *in vitro* results in less than or equal to 20% cell death, and their administration *in vivo* does not induce unwanted inflammation or other such adverse effects.

As used herein, "biodegradable" compounds are those that, when introduced into cells, are broken down by the cellular machinery or by hydrolysis into components that the cells can either reuse or dispose of without significant toxic effect on the cells (*i*.*e*., fewer than about 20 % of the cells are killed when the components are added to cells *in vitro*). The components preferably do not induce inflammation or other adverse effects *in vivo.* The chemical reactions relied upon to break down the biodegradable compounds may be uncatalyzed.

In general, the "effective amount" of an active agent or drug delivery device refers to the amount necessary to elicit the desired biological response. As will be appreciated by those of ordinary skill in this art, the effective amount of an agent or device may vary depending on such factors as the desired biological endpoint, the agent to be delivered, the composition of the encapsulating matrix, the target tissue, *etc.* For example, the effective amount of microparticles containing an antigen to be delivered to immunize an individual is the amount that results in an immune response sufficient to prevent infection with an organism having the administered antigen.

The term "surfactant" is art-recognized and herein refers to an agent that lowers the surface tension of a liquid.

The term "treating" is art-recognized and includes preventing a disease, disorder or condition from occurring in an animal which may be predisposed to the disease, disorder and/or condition but has not yet been diagnosed as having it; inhibiting the disease, disorder or condition, *e*.*g*., impeding its progress; and relieving the disease, disorder, or condition, *e*.*g*., causing regression of the disease, disorder and/or condition. Treating the disease or condition includes ameliorating at least one symptom of the particular disease or condition, even if the underlying pathophysiology is not affected, such as treating the pain of a subject by administration of an analgesic agent even though such agent does not treat the cause of the pain.

The term "targeting moiety" is art-recognized and is used herein to refer to a moiety that localizes to or away from a specific locale. Said moiety may be, for example, a protein, nucleic acid, nucleic acid analog, carbohydrate, or small molecule. Said entity may be, for example, a therapeutic compound such as a small molecule, or a diagnostic entity such as a detectable label. Said locale may be a tissue, a particular cell type, or a subcellular compartment. The targeting moiety may direct the localization of an active entity. Said active entity may be a small molecule, protein, polymer, or metal. Said active entity may be useful for therapeutic or diagnostic purposes.

The term "corresponding particle" is used herein to refer to a particle that is substantially identical to a particle to which it is compared, but typically lacking a mucoresistant surface modification (*e*.*g*., coating with a triblock copolymer). A corresponding particle may be of similar material, density, and size as the particle to which it is compared.

The term "diameter" is art-recognized and is used herein to refer to either of the physical diameter or the hydrodynamic diameter of the entity in question. The diameter of an essentially spherical particle may refer to the physical or hydrodynamic diameter. The diameter of a nonspherical particle may refer preferentially to the hydrodynamic diameter. As used herein, the diameter of a non-spherical particle may refer to the largest linear distance between two points on the surface of the particle. When referring to multiple particles, the diameter of the particles typically refers to the average diameter of the particles referred to.

A "patient," "subject," or "host" to be treated by the subject method may mean either a human or non-human animal, such as primates, mammals, and vertebrates.

These and other aspects described herein will be further appreciated upon consideration of the following Examples, which are intended to illustrate certain particular embodiments of the invention but are not intended to limit its scope, as defined by the claims.

### Examples

### Example 1 - PEG-based surfactant for engineering drug-loaded mucus-penetrating particles

The following describes a non-limiting example of a method to form a dense layer of low MW PEG on the surface of biodegradable MPP is the use of surfactants that comprise a low MW PEG moiety. An increasingly adopted surfactant in the drug delivery community is Vitamin E-PEG1k conjugate (VP1k, commonly referred to as Vitamin E TPGS), prepared by esterifying the hydrophobic D-alpha-tocopheryl acid (*i*.*e*., Vitamin E) succinate with 1 kDa PEG [1].

To test if VP1k may reduce mucoadhesion, poly(lactide-co-glycolide) (PLGA) nanoparticles were formulated by nanoprecipitation with VP1k in the aqueous phase (PLGA/VP1k); VP1k coating was confirmed by the markedly less negative surface charge of PLGA/VP1k particles compared to the highly negative surface charge of uncoated PLGA particles (Table 1).

**Table 1. Characterization of nanoparticles**

| **Particle** | **Diameter** [nm] | **ζ- Potential** [mV] |
|---|---|---|
| PS-COOH (Uncoated) | 217 ± 5 | -59 ± 4 |
| PLGA/PVA | 141 ± 9 | -1 ± 1 |
| PLGA/VP1k | 215 ± 18 | -19 ± 3 |
| PLGA/VP5k | 271 ± 10 | -8 ± 1 |
| PS-PEG | 232 ± 7 | -2 ± 1 |

The dynamics of PLGA/VP1k particles which were additionally exposed to Pluronic® F127, thereby forming F127 coated particles (PLGA/VP1K-F127; see the Methods section), in fresh human cervicovaginal mucus (CVM) collected from donors with healthy vaginal flora using multiple particle tracking was assessed [2,3]. Despite the surface PEG coverage, PLGA/VP1k-F127 particles were strongly trapped in human CVM to the same extent as uncoated polystyrene (PS) particles and PLGA particles coated with PVA, as evident by their highly constrained and non-Brownian time-lapse traces (Figures 1A- 1C).

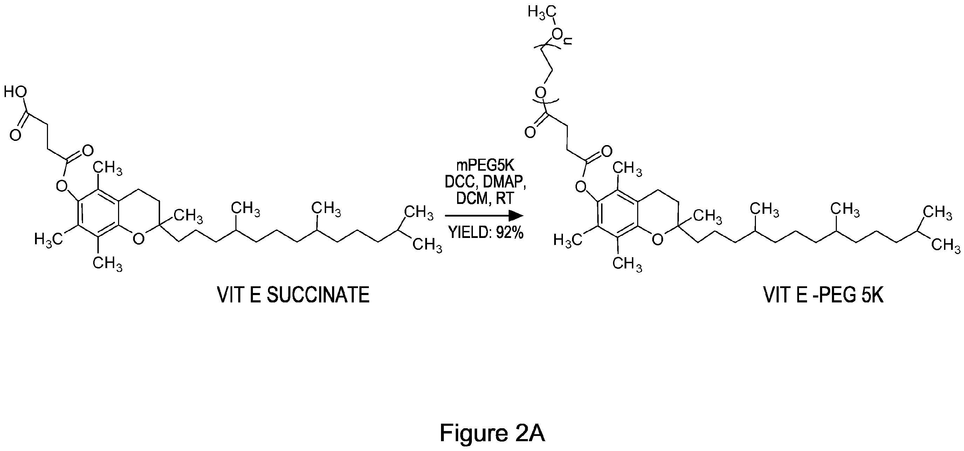

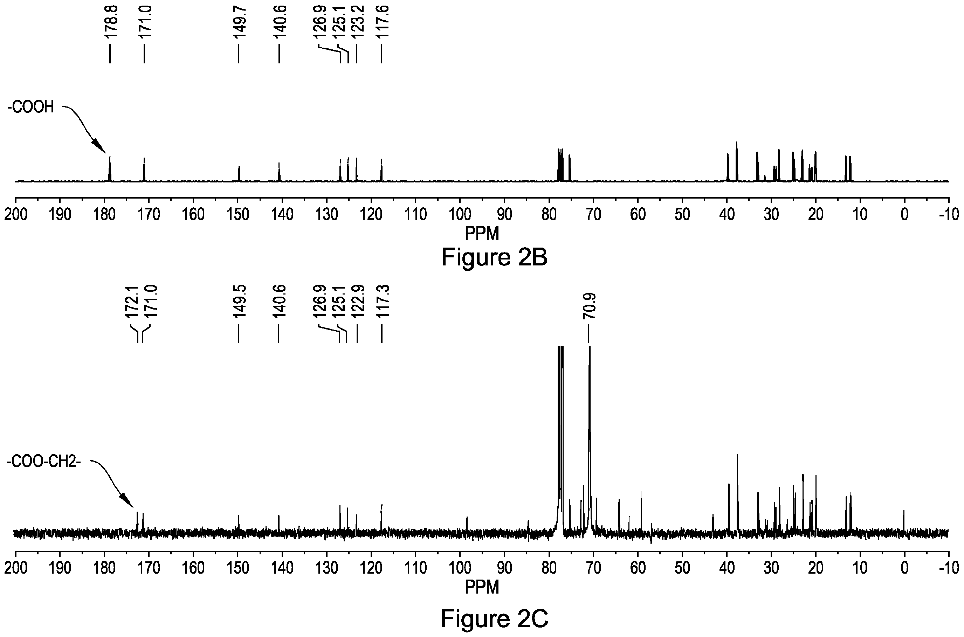

It is hypothesized that the extensive immobilization of PLGA/VP1k-F127 in CVM was due to inadequate PEG content in VP1k to adequately shield the hydrophobic PLGA core. To increase the PEG coverage, a 5 kDa PEG was conjugated to activated Vitamin E succinate (VP5k) (Figure 2A), based on previous findings that 2-5 kDa PEG coatings mediated rapid particle penetration in mucus whereas 10 kDa PEG coatings did not [4]. Successful conjugation was confirmed by ¹³C-NMR (Figure 2B). VP5k-coated PLGA nanoparticles (PLGA/VP5k) were prepared using a similar nanoprecipitation method; a greater density of surface PEG coverage by VP5k coating was evident by the roughly neutral surface charge of PLGA/VP5k particles (Table 1). In most embodiments described in this example, the PLGA/VP5K particles were additionally exposed to Pluronic® F127, thereby forming F127 coated particles (PLGA/VP5K-F127; see the Methods section).

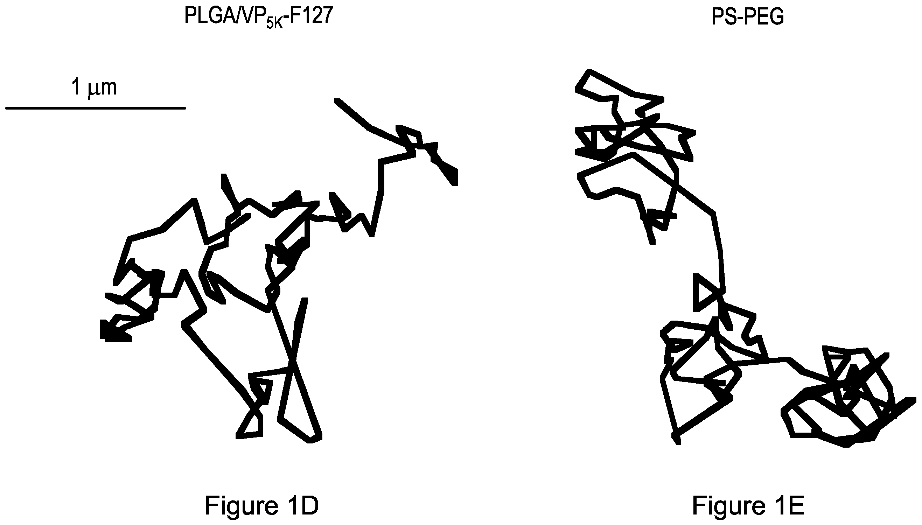

PLGA/VP5k-F127 particles rapidly penetrated CVM, as reflected by the diffusive, Brownian nature of their particle traces (Figure 1D) comparable to those of diffusive PS-PEG particles (d∼200 nm) in the same mucus samples (Figure 1E). PS particles (d∼200 nm) in the same mucus samples, which served as negative control, were extensively trapped (data not shown). To quantify particle motions, transport measurements are presented in the form of time-scale dependent ensemble mean squared displacements (<MSD>). The <MSD> of PLGA/VP5k-F127 nanoparticles was ∼210-fold higher than that for PS particles at a time scale of 1 s; the difference is statistically significant across all time scales (p < 0.01) (Figure IF). Based on the comparable <MSD> between PLGA/VP5k-F127 and PS-PEG particles, the non-covalent VP5k appeared to resist mucoadhesion to the same extent as PEG coatings generated by covalent conjugation under harsh conditions (vortex and sonication) for prolonged durations (overnight). Indeed, the fastest 50% of PLGA/VP5k-F127 particles on average penetrated CVM at speeds only 7-fold reduced compared to their theoretical speeds in water. The rapid transport of PLGA/VP5k-F127 particles was also reflected by the slope, α, of log-log plots of MSD versus time scale (α = 1 represents unobstructed Brownian transport, whereas increased obstruction to particle movement is reflected by a decrease in α): the average α was 0.64 for PLGA/VP5k-F127 particles compared to 0.31 for uncoated PS particles.

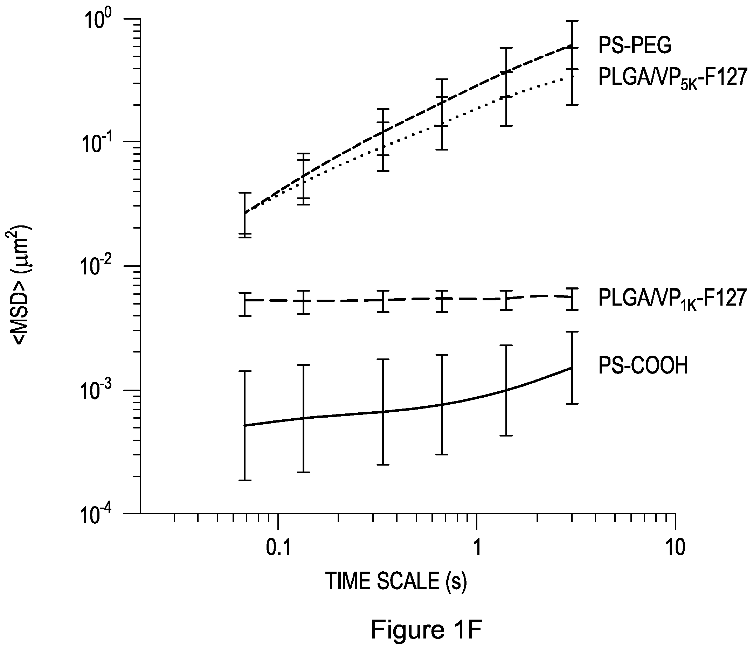

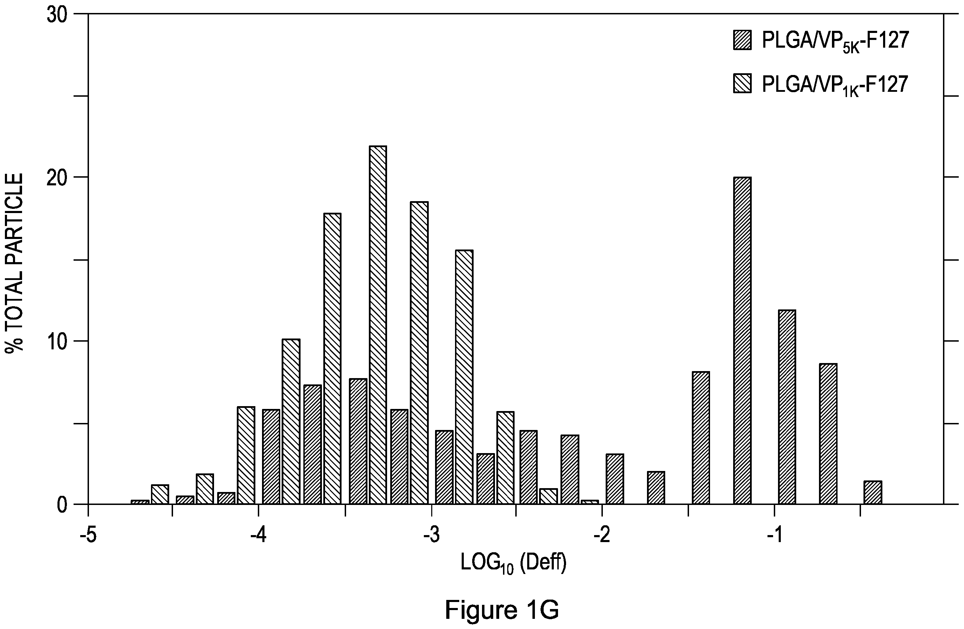

Figure 1 illustrates the effect of surfactants on the transport of PLGA particles in fresh human cervicovaginal mucus. Representative traces of (A) mucoadhesive, uncoated polystyrene particles (PS; negative control), (B) PLGA particles coated with polyvinyl-alcohol (PLGA/PVA), (C) PLGA particles coated with Vitamin E TGPS (VP1K), followed by coating with Pluronic® F127 (PLGA/VP1k-F127), (D) PLGA particles coated with a novel surfactant synthesized by conjugating methoxy-PEG5k-OH to Vitamin E succinate (VP5k), followed by coating with Pluronic® F127 (PLGA/VP5k-F127), and (E) polystyrene particles densely conjugated with 2 kDa PEG (PS-PEG), known to be muco-inert (positive control). Shown trajectories are for particles with an effective diffusivity within one SEM of the mean. Scale bars represent 1 um (micrometer) unless otherwise noted. (F): Ensemble-averaged geometric mean square displacements (<MSD>) of PLGA/VP5k-F127, PLGA/VP1k-F127, PS-COOH and PS-PEG as a function of time scale. (G): Distributions of the logarithms of individual particle effective diffusivities (D_{eff}) at a time scale of 1 s for PLGA/VP5k-F127 and PLGA/VP1k-F127 particles. Error bars represent SEM.

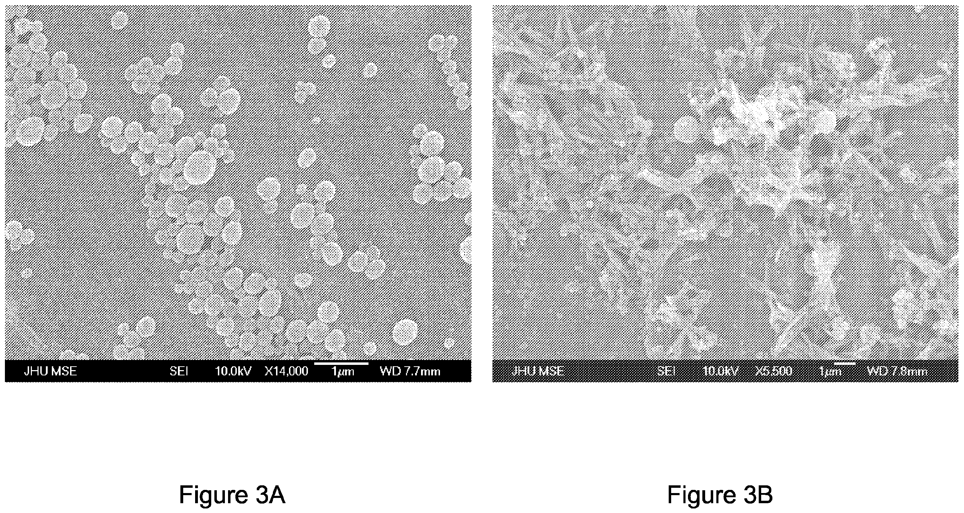

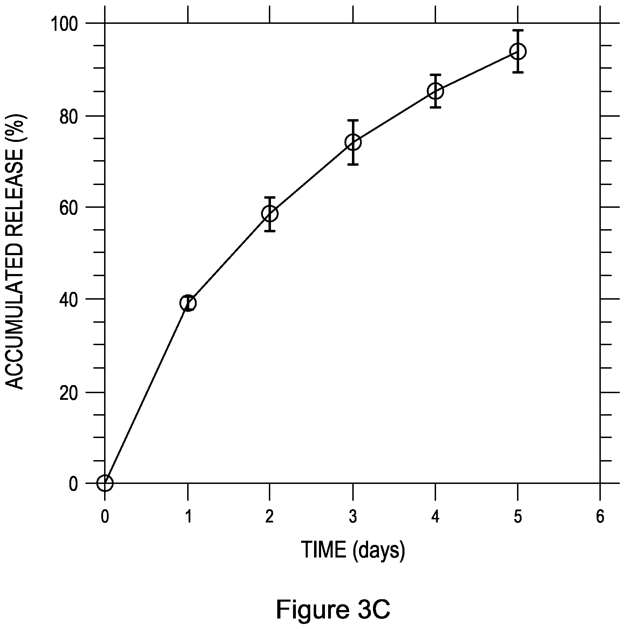

An important criterion for a suitable surfactant to formulate biodegradable drug carriers is efficient encapsulation of therapeutics. As a proof of concept, paclitaxel, a widely used anti-neoplastic agent that stabilizes microtubules and arrests tumor cells in the G2/M cell cycle phase [5], was encapsulated. Pactlitaxel-loaded particles were first prepared by coprecipitation of paclitaxel and PLGA using Pluronic® F127 as the sole surfactant (PLGA/Paclitaxel/F127), a process which generates MPP (data not shown). Electron micrographs of PLGA/Paclitaxel/F127 particles showed extensive presence of crystalline structures outside of spherical particles (presumably paclitaxel crystals formed due to its low water solubility [6]), indicating poor encapsulation (Figure 3A). Particles prepared without surfactants also exhibited similar crystalline structures outside of particles (data not shown). In contrast, paclitaxel-loaded particles prepared with VP5k (PLGA/Paclitaxel/VP5k) were free of any visible paclitaxel crystals and exhibited uniform, smooth and nonporous surfaces (Figure 3B). The paclitaxel loading was 7.9 ± 0.5% (weight of paclitaxel to weight of polymer/surfactant), with minimal burst effects and sustained release for at least 4 days (Figure 3C).

Figure 3 shows the characterization of paclitaxel-encapsulated polymeric particles. (A) SEM images of PLGA particles prepared with a commonly used surfactant (Pluronic® F127) show extensive paclitaxel crystal formation due to poor encapsulation of paclitaxel into the particles. PLGA particles prepared without surfactants exhibits similar drug crystals (data not shown). (B) SEM images of PLGA particles prepared with VP5k surfactant show no trace of paclitaxel crystals in solution. (C) Release of paclitaxel from PLGA/VP5k particles.

In summary, a novel surfactant, VP5k, was engineered that simultaneously enables highly desirable features for a biodegradable MPP drug delivery platform: (1) rapid penetration of fresh, undiluted human mucus; (2) good dispersity, low porosity and a smooth surface at the nanoscale range; (3) high loading of a small molecule drug (paclitaxel); and (4) sustained release of the drug over several days with minimal burst effects. Additional surfactants with similar functional characteristics as VP5k may be generated by conjugating PEG or other non-mucophilic polymers of an appropriate molecular weight to hydrophobic or charged molecules.

### Methods for Example 1:

*Synthesis of Vit E-PEG 5k compound:* Vit E-PEG 5k was synthesized using similar method described previously. Briefly, vitamin E succinate (0.65 g, 1.0 eq) was dissolved in dichloromethane (20 mL) in 50 ml round type flask, and methoxy polyethylene glycol (5000g/mol, 7.334 g, 1.2 eq.) was added to the mixture. After PEG was dissolved, DMAP (4-dimethylaminopyridine; 15 mg, 0.1 equivalents) was added into the flask followed by addition of DCC (N,N'-dicyclohexylcarbodiimide, 0.278 g, 1.1 equivalents.) The reaction mixture was stirred at room temperature overnight, Buchner filtered, and the filtrate was concentrated under reduced pressure to obtain crude product. The crude product was dissolved in ultrapure water at 5 % (w/v). To eliminate DCC and unreacted Vit E Succinate, both insoluble in water, the crude product was subjected to centrifugation (25k, 20 min, 2 times, Beckman Coulter) and further filtered with filter unit (0.2 micron). The final pure product yield was 92 %.

*Characterization of Vit E-PEG 5k compound:* Conjugation of mPEG to Vit E Succinate was confirmed by 13-C-NMR (400MHz, Bruker). Carbonyl carbon of -COOH of Vit E Succinate generates a signal at 178.8 ppm, while the signal of same carbonyl carbon of Vit E-PEG 5k compound shifted to 172.2 ppm, which refers to the conjugation of mPEG to Vit E Succinate. The signal of the second carbonyl carbon at 171.0 ppm, and aromatic carbons signals located between 115 ppm and 150 ppm in both reactant and product remained unchanged. Also, -OCH₂- groups of mPEG unit in VitE-PEG 5k gave a very intense signal at 70.9 ppm.

*Preparation of doxorubicin labeled PLGA nanoparticles & their characterization:* For visualizing particles in cervicovaginal mucus, poly(lactide-co-glycolide) (PLGA; M.W. 11,000 Da, 50:50) (Alkermes Inc., Cambridge, MA) was labeled with doxorubicin (NetQem, Durham, NC), used as a fluorescent marker. Dox conjugated nanoparticles were formulated by solvent diffusion technique. Briefly, 20 mg of the polymer was dissolved in 1 mL of acetonitrile, and added dropwisely into 36 mL of 1.65 % Vit E-PEG-1k or Vit E-PEG-5K. After the volatile organic solvent was removed with stirring for 3 hr in well circulated hood, the particles were collected by centrifugation at 10 k rpm (Avanti J-25 centrifuge, Beckman Coulter, Inc., Fullerton, CA) for 20 min, washed twice and resuspended in 0.2 mL of ultrapure water, thus forming PLGA/VP1K and PLGA/VP5K particles, respectively. Particle suspension was split into two equal volumes. 100 ul of ultrapure water was added into first part (PLGA/VP1K and PLGA/VP5K particles) while 200 ul of 1 % Pluronic® F127 (BASF) was added into second part (*e*.*g*., thereby forming PLGA/VP1K-F127 and PLGA/VP5K-F127 particles, respectively, from the PLGA/VP1K and PLGA/VP5K particles). Both suspensions were incubated at lowest speed of vortex for 30 min. ζ-Potential were determined by dynamic light scattering and laser Doppler anemometry, respectively, using a Zetasizer Nano ZS90 (Malvern Instruments, Southborough, MA) (see Table 2). F127 coated and uncoated PLGA-Dox/VP1k nanoparticles were formulated with the same methodology as mentioned above.

**Table 2. ζ-Potentials for PLGA/VP1K, PLGA/VP1K-F127, PLGA/VP5K, and PLGA/VP5K-F127 particles**

| | PLGA/VP1K | PLGA/VP1K-F127 | PLGA/VP5K | PLGA/VP5K-F127 |
|---|---|---|---|---|
| ζ-Potential | -19 +/- 3 mV | 7 +/-1mV | -8 +/- 1mV | -4 +/-1mV |

### References for Example 1:

1. Mu, L. and S.S. Feng, Vitamin E TPGS used as emulsifier in the solvent evaporation/extraction technique for fabrication of polymeric nanospheres for controlled release of paclitaxel (Taxol (R)). Journal of Controlled Release, 2002. 80(1-3): p. 129-144.
2. Apgar, J., Y. Tseng, E. Fedorov, M.B. Herwig, S.C. Almo, and D. Wirtz, Multiple-particle tracking measurements of heterogeneities in solutions of actin filaments and actin bundles. Biophys J, 2000. 79(2): p. 1095-106.
3. Suh, J., M. Dawson, and J. Hanes, Real-time multiple-particle tracking: applications to drug and gene delivery. Adv Drug Deliv Rev, 2005. 57(1): p. 63-78.
4. Wang, Y.Y., S.K. Lai, J.S. Suk, A. Pace, R. Cone, and J. Hanes, Addressing the PEG mucoadhesivity paradox to engineer nanoparticles that "slip" through the human mucus barrier. Angew Chem Int Ed Engl, 2008. 47(50): p. 9726-9.
5. Bhalla, K.N., Microtubule-targeted anticancer agents and apoptosis. Oncogene, 2003. 22(56): p. 9075-86.
6. Singla, A.K., A. Garg, and D. Aggarwal, Paclitaxel and its formulations. Int J Pharm, 2002. 235(1-2): p. 179-92.

### Example 2:

### Simple and safe biodegradable nanoparticles that easily penetrate human mucus

It was recently demonstrated that covalently coating particles with a high density of low MW poly(ethylene glycol) (PEG), a hydrophilic and uncharged polymer widely used in pharmaceuticals, can reduce particle affinity to mucus constituents analogous to the surfaces of some viruses that infect mucosal tissues [1]. These densely coated particles were able to rapidly penetrate fresh, undiluted human mucus at speeds only a few-fold reduced compared to their speeds in water [1, 2]. Nevertheless, current methods to produce mucus-penetrating particles (MPP) involve the use of either PEG-containing block copolymers [3, 4] or covalent PEGylation of pre-fabricated particles [1, 2]; both methods lead to particles composed of new chemical entities as defined by the FDA. The use of these systems imposes a complicated, expensive and time-consuming path through the FDA regulatory process, including extensive clinical toxicity and safety studies. This reality has strongly limited the commercial development of nanoparticle-based drug delivery systems. It was sought to develop a simple non-covalent coating process to produce MPP composed entirely of GRAS (Generally Regarded As Safe by the FDA) materials. It is hypothesized that uncharged amphiphilic GRAS materials, such as triblock copolymers of poly(ethylene glycol)-poly(propylene oxide)-poly(ethylene glycol) (PEG-PPO-PEG; known as Pluronics®), may readily coat hydrophobic particle surfaces. The hydrophobic segments of such materials may adhere tightly to the particle core, leaving a dense brush of uncharged, hydrophilic segments protruding from the particle surface that minimizes mucoadhesion.

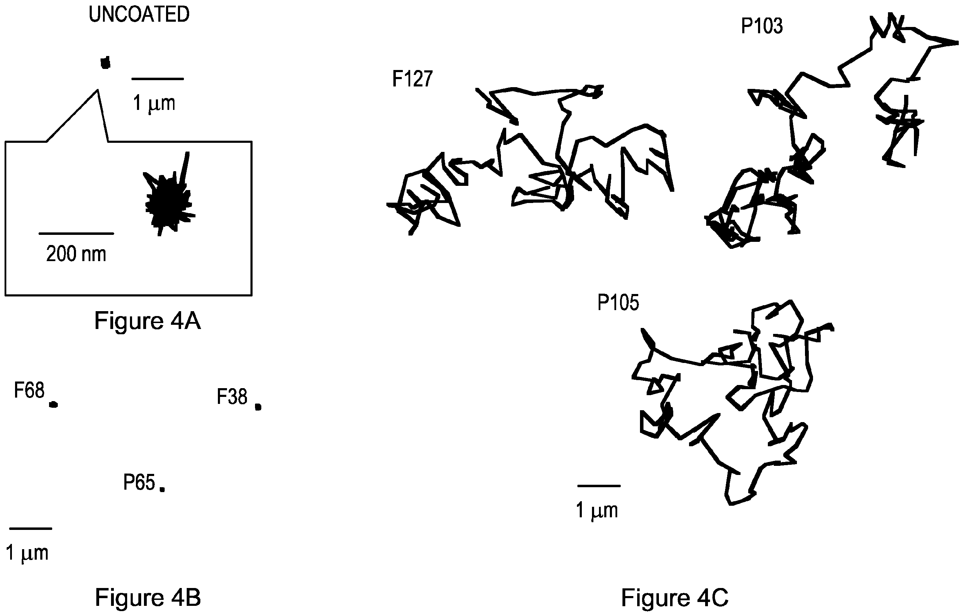

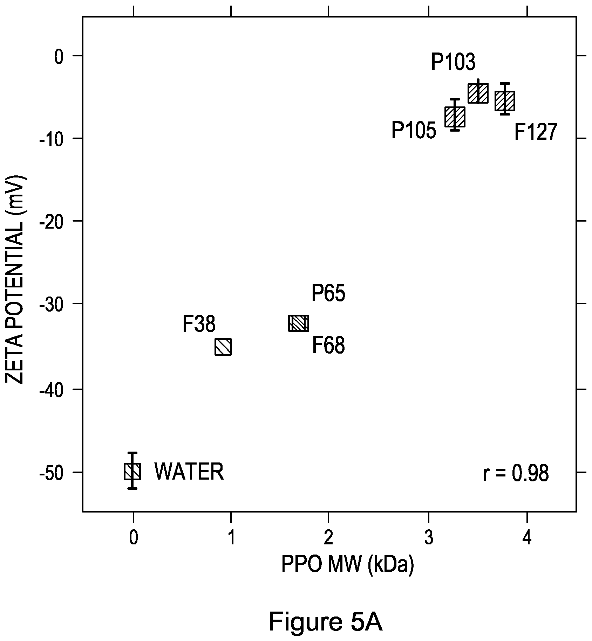

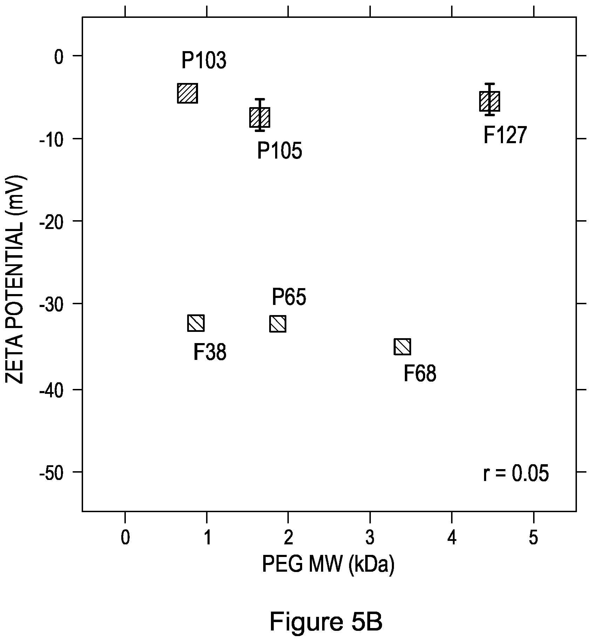

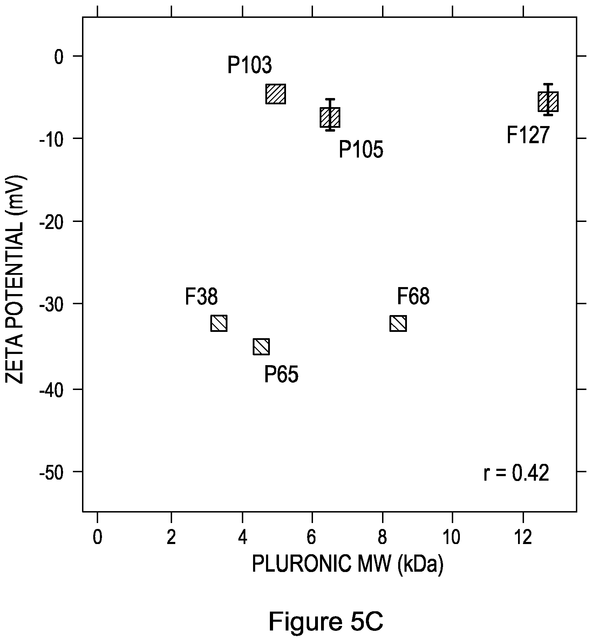

Pluronics® are commercially available in a variety of MW and PPO/PEG ratios, and different Pluronics® have been adopted for various biomedical applications [5, 6, 7]. Pluronics® can transform mucoadhesive polymeric nanoparticles into MPP were identified. As a proof-of-concept, nanoparticles composed of the GRAS material poly(lactide-co-glycolide) (PLGA) with a covalently tagged fluorophore were formed and incubated with Pluronic® F38, P65, P103, P105, F68 and F127 (listed in order of increasing MW), followed by purification. The transport dynamics in fresh, undiluted human cervicovaginal mucus (CVM) were observed. Uncoated PLGA nanoparticles are negatively charged at neutral pH, and extensively immobilized in CVM (Figure 4A). Three of the Pluronics® (F38, P65 and F68) tested did not enhance the transport of PLGA particles, as evident from their highly constrained, non-Brownian time-lapse traces (Figure 4B). In contrast, coating PLGA particles with P103, P105 or F127 enabled them to readily penetrate CVM, as evident from their diffusive, Brownian trajectories that covered large distances over the course of 20 s movies (Figure 4C). The effectiveness of the Pluronic® coatings was critically dependent on the MW of the PPO segment (Figure 4E), perhaps because adhesive interactions between short PPO segments and PLGA are inadequate to anchor a dense brush of Pluronic® molecules (and consequently PEG) onto the particle surface. To confirm whether PPO MW correlates with the density of Pluronic® surface coverage, the ζ-potential (surface charge) of PLGA particles incubated in the various Pluronics® was measured. P103, P105, and F127 all have PPO MW ≥ 3 kDa, and produced coated particles with a ζ-potential > - 8 mV (Figure 5A); PEG-coatings have been previously found to effectively shield the mucoadhesive core of latex particles result in a particle ζ-potential value > -10mV [2]. In contrast, PLGA nanoparticles incubated in F38, P65, and F68, each of which possess PPO segments with MW < 3 kDa, exhibited surface charges between -30 to -35 mV, indicating some but inadequate surface coverage by the neutrally-charged PEG segments. There was no correlation between the Pluronic® coating density and either the MW of the PEG segments or total Pluronic® MW (Figures 5B and 5C). The near neutral surface charges for P103-, P105- and F127-coated particles were also observed 24 hr after particle synthesis, suggesting the coating is stable at least over that duration (data not shown).

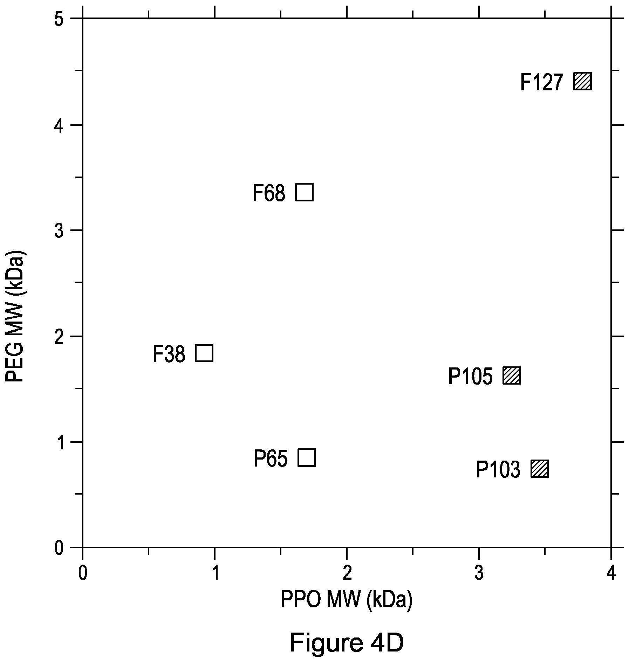

Figure 4 shows the transport behaviors of uncoated and Pluronic®-coated PLGA particles in fresh human cervicovaginal mucus (CVM). (A), (B), (C): Representative trajectories in fresh human CVM of (A) uncoated PLGA particles, (B) particles coated with low PPO MW Pluronic® (F68, F38 or P65) and (C) particles coated with high PPO MW Pluronic® (F127, P103 or P105), respectively. (D): various Pluronics® with different MW of PPO and PEG segments. Filled symbols indicate mucus-penetrating particle formulations, while open symbols indicate mucoadhesive formulations.

Figure 5 shows muco-inert vs. mucoadhesive behavior of PLGA particles coated with various Pluronics® (F38, P65, P103, P105, F68 and F127) in fresh human CVM. (A), (B), (C): Correlation between the zeta potential of Pluronic®-coated PLGA particles and the MW of the (A) PPO segment, (B) PEG segment or (C) entire Pluronic® molecule. In (A), "Water" indicates the zeta potential of uncoated PLGA particles made in water. Filled symbols indicate MPP formulations, while open symbols indicate mucoadhesive formulations. Data represent observations of particles from at least two different batches in five different mucus samples, with all formulations tested in the same mucus samples. r represents the correlation coefficient.

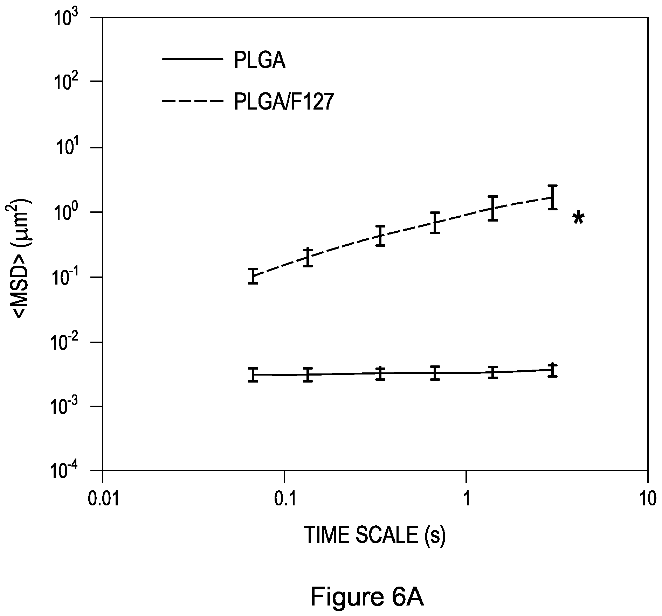

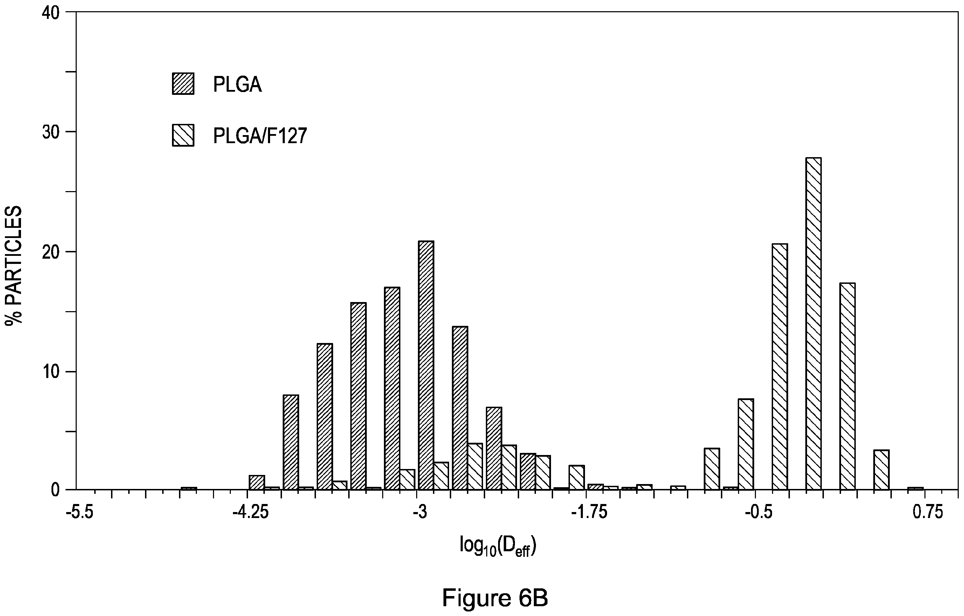

Pluronic® F127 is one of the most commonly used Pluronics® for pharmaceutical applications [8-9]; subsequent investigations focused on F127. To quantify the speeds of F127-coated PLGA nanoparticles (PLGA/F127) in mucus, the motions of PLGA/F127 were analyzed using multiple particle tracking, a powerful biophysical technique that allows quantitative measurements of hundreds of individual particles. The time-scale dependent ensemble mean squared displacement (<MSD>) of PLGA/F127 was 280-fold higher than that for uncoated PLGA particles (PLGA) at a time scale of 1 s, and the difference in <MSD> was statistically significant across all time scales (Figure 6A). Few, if any, PLGA/F127 nanoparticles were trapped in mucus compared to PLGA (Figure 6B). The difference in the transport rates of PLGA and PLGA/F127 nanoparticles was also reflected by the slope, α, of log-log plots of particle <MSD> versus time scale (α = 1 represents unobstructed Brownian transport, whereas increasing obstruction to particle movement is reflected by a decrease in α): the average α was 0.69 for PLGA/F127 compared to 0.04 for PLGA. Importantly, PLGA/F127 nanoparticles were slowed only ∼10-fold in CVM compared to their theoretical speeds in water, whereas PLGA nanoparticles were slowed ∼4000-fold (Table 3). The similar speeds of particles coated with Pluronic® F127 and surface conjugated with low MW PEG [1-2] suggest that the non-covalent Pluronic® coating shields adhesive particle surfaces as efficiently as do covalent PEG coatings.

**Table 3. Characterization of various uncoated and F127-coated nanoparticles and ratios of the ensemble average diffusion coefficients in CVM (Dₘ) compared to in water (D_{w}).**

| **Formulation** | **Diameter, nm** | **ζ-potential, mV** | ***D_{w}*/*Dₘ*^{†}** |
|---|---|---|---|
| PLGA | 110 ± 4 | - 50 ± 2 | 3800 |
| PLGA/F127 | 138 ± 2 | - 5 ± 2 | 10 |
| PCL | 122 ± 2 | - 6 ± 2 | 2400 |
| PCL/F127 | 135 ± 5 | - 1 ± 1 | 6 |
| PS | 194 ± 6 | - 46 ± 1 | 4000 |
| PS/F127 | 216 ± 2 | -4 ± 1 | 4 |

| | | | |
|---|---|---|---|
| ^{†} Effective diffusivity values are calculated at a time scale of 1 s. *D*_{w} is calculated from the Stokes-Einstein equation. ^{†} Effective diffusivity values are calculated at a time scale of 1 s. *D*_{w} is calculated from the Stokes-Einstein equation. | | | |

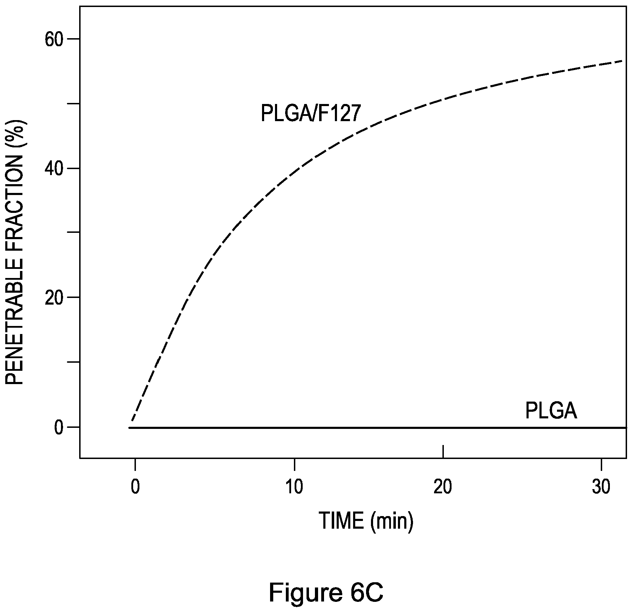

Figure 6 shows the transport of F127-coated PLGA particles and uncoated particles in human CVM. (A): Ensemble-averaged geometric mean square displacements (<MSD>) as a function of time scale. (B): Distributions of the logarithms of individual particle effective diffusivities (D_{eff}) at a time scale of 1 s. Data represent at least three experiments, with n ≥ 138 and average n = 155 and 147 for PLGA and PLGA/F127, respectively. * denotes statistically significant difference across all time scales (p < 0.05). (C): The estimated fraction of particles predicted to be capable of penetrating a 30 µm thick mucus layer over time).

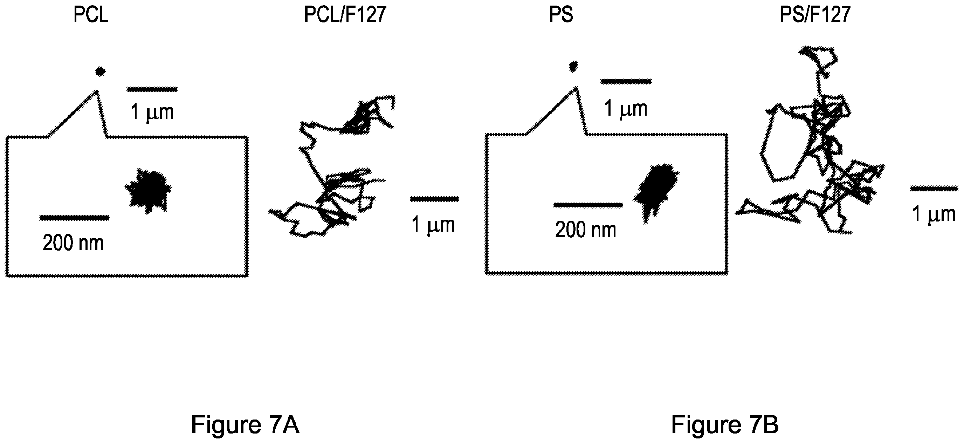

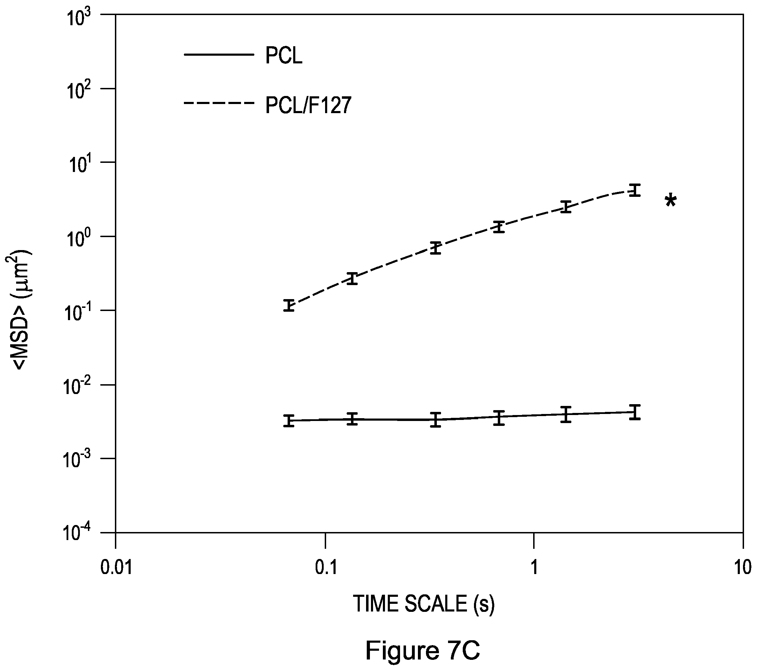

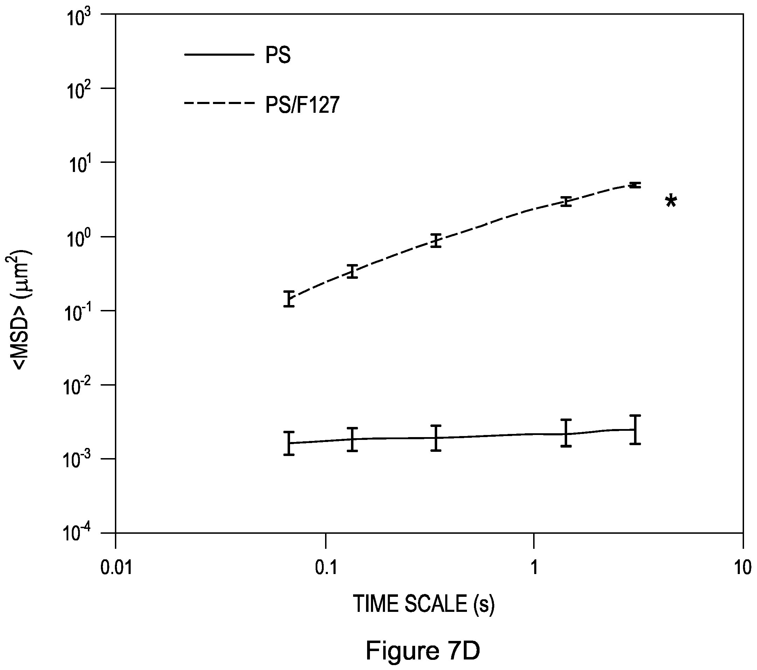

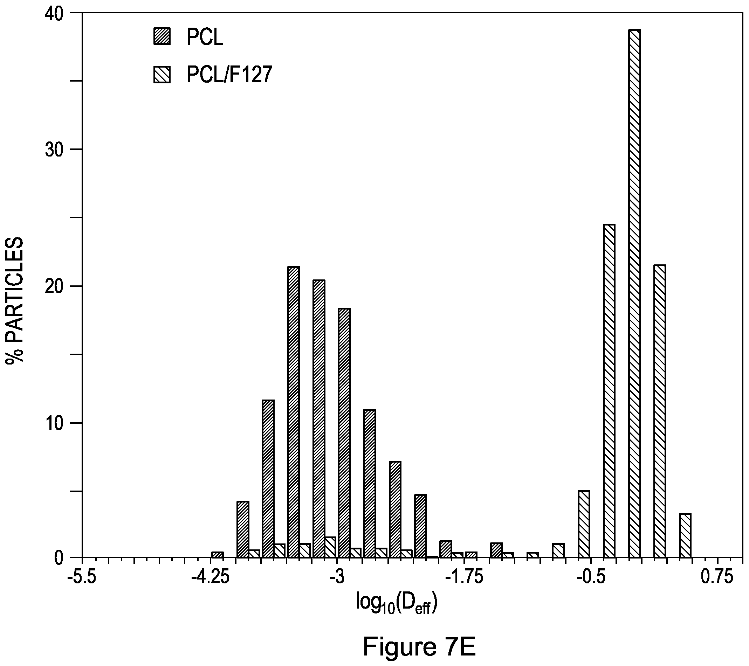

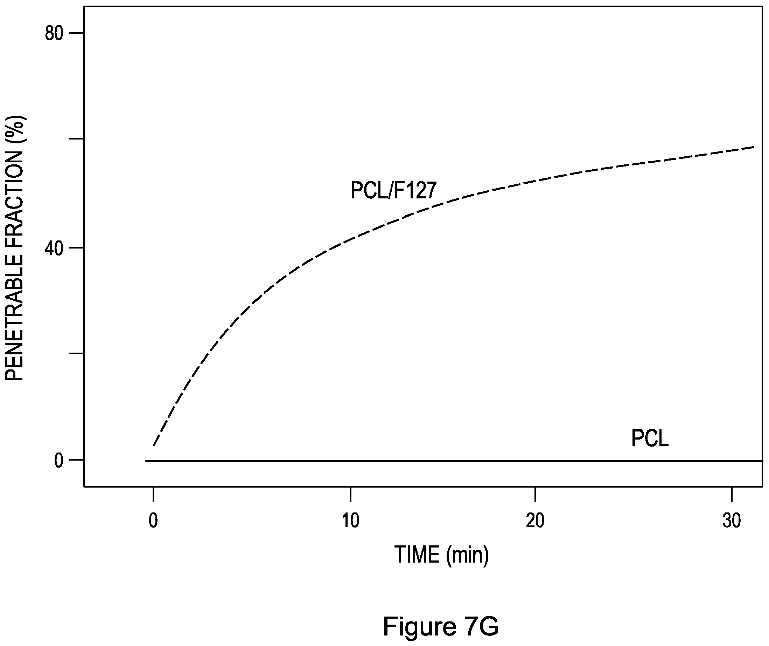

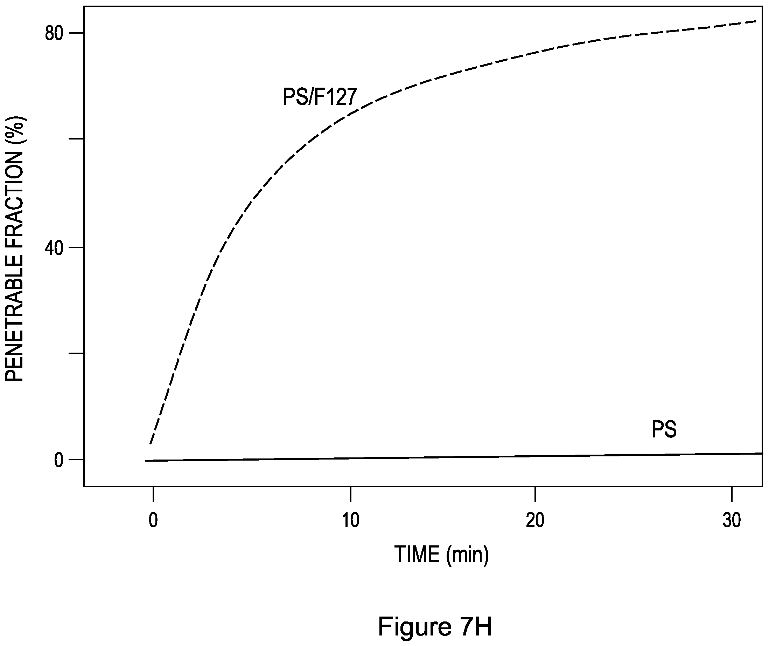

To investigate whether Pluronic® can also transform particles composed of other mucoadhesive polymers into MPP, particles composed of the widely used hydrophobic poly(ε-caprolactone) (PCL) polymer as well as a generic hydrophobic material, polystyrene (PS; also known as latex), coating both with Pluronic® F127 (producing PCL/F127 and PS/F127, respectively) were tested. Similar to the results with PLGA and PLGA/F127, the time-lapse traces of uncoated PCL and PS particles were highly constrained and non-Brownian, while those of PCL/F127 and PS/F127 were Brownian (Figures 7A and 7B). PCL/F127 particles exhibited a 500-fold higher <MSD> than PCL particles (at a time scale of 1 s; p < 0.005) (Figure 7C). Both the average effective diffusivity (D_{eff}) of PCL/F127 in CVM (∼3.5-fold lower compared to that in water) and the distribution of particle speeds (Figure 7E) agreed well with the transport rates achieved by PLGA/F127. Likewise, the <MSD> of PS/F127 was 1100-fold higher than that for PS (p < 0.01), and the average D_{eff} of PS/F127 was only 4-fold lower than that for the same particles in pure water (Figures 7D AND 7E). Based on the speeds achieved, the majority of F127-coated particles, regardless of the core material *(i.e.,* PLGA vs. PCL vs. PS), are expected to penetrate physiologically-thick mucus layers in minutes (Figure 6C, 7G, and 7H).

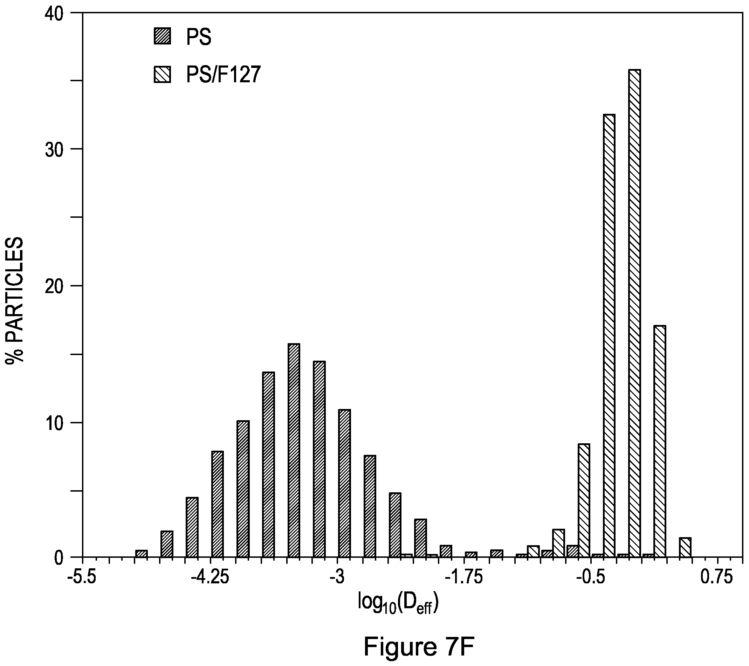

Figure 7 shows the transport of F127-coated PCL and PS particles and uncoated particles in human CVM. (A), (B): Representative trajectories of uncoated particles and particles coated with Pluronic® F127 in CVM. (C), (D): Ensemble-averaged geometric mean square displacements (<MSD>) as a function of time scale. (E), (F): Distributions of the logarithms of individual particle effective diffusivities (D_{eff}) at a time scale of 1 s. Data represent at least three experiments, with n ≥ 111 and average n = 118 and 153 for PCL and PCL/F127, respectively, and n ≥ 150 and average n = 185 and 152 for PS and PS/F127, respectively. * denotes statistically significant difference across all time scales (p < 0.05). (G), (H): The estimated fraction of particles predicted to be capable of penetrating a 30 µm thick mucus layer over time.

The Pluronic® coating process reported here, which transforms conventional mucoadhesive particles into MPP, offers numerous advantages for drug delivery applications to mucosal surfaces. First, Pluronic® has an extensive safety profile and has been used since the 1950s [5] in many commercially available products, including drug delivery devices such as Elitek® (intravenous infusion) [10], Zmax® (oral suspension) [11] and Oraqix® (periodontal gel) [12]. Combining Pluronic® with other GRAS materials may, therefore, produce mucus-penetrating drug delivery platforms that are likely to be safe in humans and greatly reduce the time and costs for clinical development. Second, since this method involves only a short incubation of pre-fabricated particles with Pluronic®, the formulation process of the drug-loaded particle core remains unchanged. The simplicity of the coating process may accelerate economical and scalable translational development of the MPP technology. Third, tailored release profiles and high encapsulation efficiencies may be achieved for a wide array of cargo therapeutics simply by selecting an appropriate GRAS material, with optimal degradation kinetics and polymer-drug affinity, for the particle core. The freedom to choose core polymers that degrade on the same time scale as drug release may help minimize the potential buildup of unwanted polymers in the body, as can occur with repeated administration of carriers that release drug quickly but are composed of slowly degrading polymers [13]. Fourth, Pluronic® coatings may also facilitate rapid particle penetration at other mucosal surfaces, since human CVM possesses biochemical content and rheological properties similar to those of mucus fluids derived from the eyes, nose, lungs, gastrointestinal tract and more [1]. Indeed, a Pluronic® F127 coating markedly improved the transport of polymeric particles in both sputum expectorated by cystic fibrosis patients as well as mucus collected via surgery from the nasal cavity of patients with chronic sinusitis.

Drug carriers composed of GRAS materials, such as PLGA or PCL, are extensively immobilized in human mucus and quickly eliminated from mucosal surfaces. It was shown in this example that, in some cases, Pluronic® molecules enable these particles to rapidly penetrate human mucus secretions. Enhanced mucus penetration is expected to facilitate prolonged retention and more uniform distribution of drug carriers at mucosal surfaces, leading to improved pharmacokinetics and therapeutic efficacy [14].

### Methods for Example 2:

### Preparation and characterization of Pluronic®-coated nanoparticles:

Doxorubicin (NetQem, Durham, NC), with excitation/emission maxima at 480/550 nm, was chemically conjugated to PLGA (MW 11,000 Da, 50:50) (Alkermes Inc., Cambridge, MA) and PCL (MW 14,000 Da) (Polymer Source Inc., Dorval, QC, Canada) as previously described [15]. Fluorescent nanoparticles were prepared by using a nanoprecipitation method [16]. Briefly, 10 mg of the labeled polymer was dissolved in 1 mL of tetrahydrofuran, and added dropwise into 40 mL of aqueous solution. After stirring for 3 hr to remove the organic solvent, the particles were collected by centrifugation at 14,636 ×g (Avanti J-25 centrifuge, Beckman Coulter Inc., Fullerton, CA) for 20 min and washed twice. For particles coated with Pluronic® (BASF, Ludwigshafen, Germany), ultrapure water was replaced by 0.1% Pluronic® aqueous solution during the washing steps, and the particles were resuspended in 0.4 mL of 1% Pluronic® solution. The particle suspensions were subsequently centrifuged at 92 ×g (MicroA Marathon centrifuge, Fisher Scientific, Pittsburgh, PA) for 2 min to remove any potential aggregates, and the supernatants (containing non-aggregated PLGA/Pluronic® particles) were purified by size exclusion chromatography. Fluorescent carboxyl-modified polystyrene particles 200 nm in size (Molecular Probes, Eugene, OR) were similarly coated with Pluronic® as described above. Size and α-potential were determined by dynamic light scattering and laser Doppler anemometry, respectively, using a Zetasizer Nano ZS90 (Malvern Instruments, Southborough, MA).

### Human cervicovaginal mucus (CVM) collection:

CVM was collected as previously described [1, 17]. Briefly, undiluted cervicovaginal secretions from women with normal vaginal flora were obtained using a self-sampling menstrual collection device following a protocol approved by the Institutional Review Board of the Johns Hopkins University. The device was inserted into the vagina for ∼30 s, removed, and placed into a 50 mL centrifuge tube. Samples were centrifuged at 1,000 rpm for 2 min to collect the mucus secretions.

### Multiple particle tracking:

Particle transport rates were measured by analyzing trajectories of yellow-green or red fluorescent particles, recorded using a silicon-intensified target camera (VE-1000, Dage-MTI, Michigan, IN) mounted on an inverted epifluorescence microscope (Zeiss, Thornwood, NY) equipped with a 100x oil-immersion objective (N.A., 1.3) and the appropriate filters. Experiments were carried out in custom-made chamber slides, where diluted particle solutions (0.0082% wt/vol) were added to 20 µL of fresh mucus to a final concentration of 3 % v/v (final particle concentration, 8.25 x 10 -7 wt/vol) and incubated at 37°C for 2 h before microscopy. Trajectories of n ≥ 100 particles were analyzed for each experiment, and at least three independent experiments were performed for each condition. Movies were captured with MetaMorph software (Universal Imaging, Glendale, WI) at a temporal resolution of 66.7 ms for 20 s. The tracking resolution was 10 nm, as determined by tracking the displacements of particles immobilized with a strong adhesive [1]. The coordinates of nanoparticle centroids were transformed into time-averaged MSD, calculated as <Δr2(τ)> = [x(t+τ) - x(t)]² + [y(t+τ) - y(t)]², where x and y represent the nanoparticle coordinates at a given time and τ is the time scale or time lag. Distributions of MSDs and effective diffusivities were calculated from this data, as demonstrated previously [1, 18-19]. Particle penetration into a mucus layer was modelled using Fick's second law and diffusion coefficients obtained from tracking experiments [3].

### References for Example 2:

1. Lai, S.K., et al., Rapid transport of large polymeric nanoparticles in fresh undiluted human mucus. Proc Natl Acad Sci U S A, 2007. 104(5): p. 1482-7.
2. Wang, Y.Y., et al., Addressing the PEG mucoadhesivity paradox to engineer nanoparticles that "slip" through the human mucus barrier. Angew Chem Int Ed Engl, 2008. 47(50): p. 9726-9.
3. Tang, B.C., et al., Biodegradable polymer nanoparticles that rapidly penetrate the human mucus barrier. Proc Natl Acad Sci U S A, 2009. 106(46): p. 19268-73.
4. Cu, Y. and W.M. Saltzman, Controlled surface modification with poly(ethylene)glycol enhances diffusion of PLGA nanoparticles in human cervical mucus. Mol Pharm, 2009. 6(1): p. 173-81.
5. Emanuele, R.M., FLOCOR: a new anti-adhesive, rheologic agent. Expert Opin Investig Drugs, 1998. 7(7): p. 1193-200.
6. Batrakova, E.V. and A.V. Kabanov, Pluronic block copolymers: evolution of drug delivery concept from inert nanocarriers to biological response modifiers. J Control Release, 2008. 130(2): p. 98-106.
7. Rodeheaver, G.T., et al., Pluronic F-68: a promising new skin wound cleanser. Ann Emerg Med, 1980. 9(11): p. 572-6.
8. Escobar-Chavez, J.J., et al., Applications of thermo-reversible pluronic F-127 gels in pharmaceutical formulations. J Pharm Pharm Sci, 2006. 9(3): p. 339-58.
9. Dumortier, G., et al., A review of poloxamer 407 pharmaceutical and pharmacological characteristics. Pharm Res, 2006. 23(12): p. 2709-28.
10. Pui, C.H., Rasburicase: a potent uricolytic agent. Expert Opin Pharmacother, 2002. 3(4): p. 433-42.
11. Lo, J.B., et al., Formulation design and pharmaceutical development of a novel controlled release form of azithromycin for single-dose therapy. Drug Dev Ind Pharm, 2009. 35(12): p. 1522-9.
12. Donaldson, D., et al., A placebo-controlled multi-centred evaluation of an anaesthetic gel (Oraqix) for periodontal therapy. J Clin Periodontol, 2003. 30(3): p. 171-5.
13. Fu, J., et al., New polymeric carriers for controlled drug delivery following inhalation or injection. Biomaterials, 2002. 23(22): p. 4425-33.
14. Lai, S.K., Y.Y. Wang, and J. Hanes, Mucus-penetrating nanoparticles for drug and gene delivery to mucosal tissues. Adv Drug Deliv Rev, 2009. 61(2): p. 158-71.
15. Yoo, H.S., et al., Biodegradable nanoparticles containing doxorubicin-PLGA conjugate for sustained release. Pharm Res, 1999. 16(7): p. 1114-8.
16. Farokhzad, O.C., et al., Targeted nanoparticle-aptamer bioconjugates for cancer chemotherapy in vivo. Proc Natl Acad Sci U S A, 2006. 103(16): p. 6315-20.
17. Boskey, E.R., et al., A self-sampling method to obtain large volumes of undiluted cervicovaginal secretions. Sex Transm Dis, 2003. 30(2): p. 107-9.
18. Dawson, M., D. Wirtz, and J. Hanes, Enhanced viscoelasticity of human cystic fibrotic sputum correlates with increasing microheterogeneity in particle transport. J Biol Chem, 2003. 278(50): p. 50393-401.
*19.* Suh, J., M. Dawson, and J. Hanes, Real-time multiple-particle tracking: applications to drug and gene delivery. Adv Drug Deliv Rev, 2005. 57(1): p. 63-78.

### Example 3:

### Addition of Free Pluronic® to Mucus

The addition of free Pluronic® to mucosal tissues may improve the transportation of particles through mucosal tissues as compared to the transport of particles through mucosal tissues without the presence of Pluronic®. In some cases, the Pluronic® may increase particle transport by masking hydrophobic domains in mucins that may trap mucoadhesive particles instead of coating the particles surface.

To demonstrate that the addition of Pluronic® can improve transport of otherwise mucoadhesive particles in human mucus, free Pluronic® F127 was added to human cervicovaginal mucus, and the consequent particle mobility was quantified. Pluronic® F127 solutions of various concentrations were added at 1% v/v to human cervicovaginal mucus samples, thereby obtaining final concentrations of 0.0001%, 0.01%, or 1% w/v (*i*.*e*., 0.001, 0.1 and 10 mg/mL) Pluronic® in mucus. As control experiments, the same volume of saline was added to different aliquots of the same mucus samples. After the addition of Pluronic®, fluorescent 200 nm carboxyl-modified polystyrene (PS) particles were administered to the mucus samples, and the mucus was incubated at 37 °C for 2 h before microscopy. PS particles added to saline-treated, 0.0001%, 0.01%, or 1% Pluronic®-treated mucus are referred to as PS_{0% F127}, PS_{0.0001% F127}, PS_{0.01% F127}, and PS_{1% F127}, respectively.

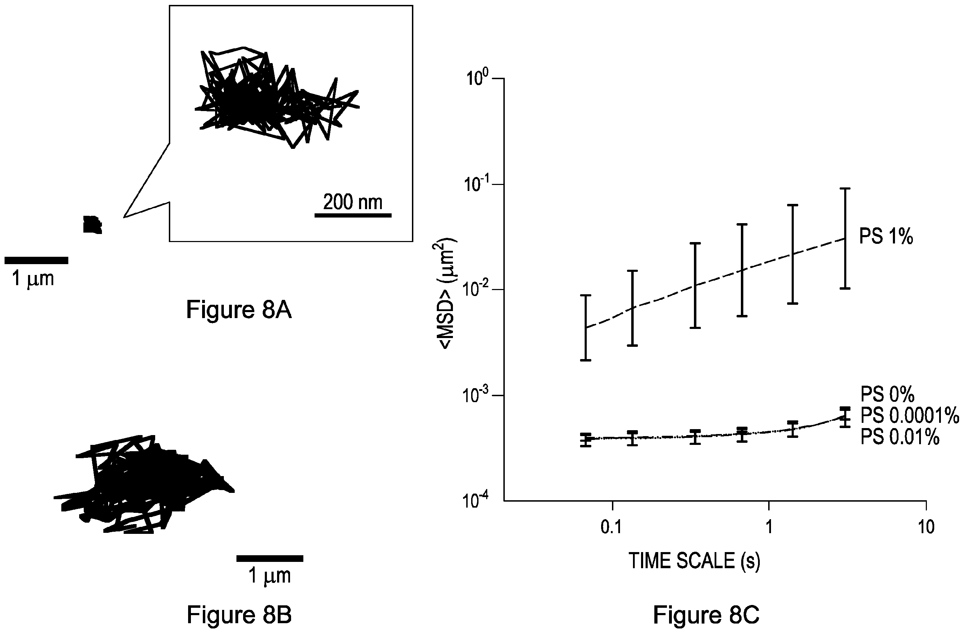

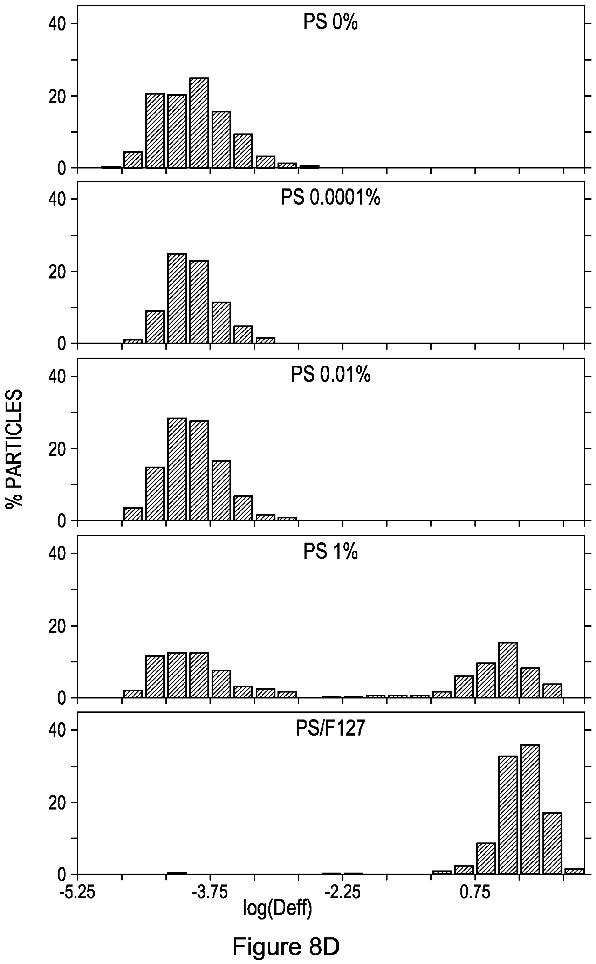

The diffusion of PS in saline- or Pluronic®-treated mucus gels using multiple particle tracking was analyzed. Similar to previous findings [1], the time-lapse traces of PS_{0% F127} were highly constrained and non-Brownian (Figure 8A), as were both PS_{0.0001% F127} and PS_{0.01% F127}. However, PS_{1%} exhibited much more diffusive trajectories that probed much larger distances (Figure 8B). To quantify particle motions, transport measurements in the form of time-scale dependent ensemble mean squared displacements (<MSD>) are presented. The <MSD> of PS_{1% F127} was ∼40-fold higher than that for PS_{0% F127}, PS_{0.0001% F127}, and PS_{0.01% F127} at a time scale of 1 s, and the difference in <MSD> was statistically significant across all time scales (Figure 8C). The difference in the particle transport rates was also reflected by the slope, α, of log-log plots of <MSD> versus time scale (α = 1 represents unobstructed Brownian transport, whereas increasing obstruction to particle movement is reflected by a decrease in α): the average α was 0.49 for PS_{1% F127} compared to 0.14, 0.15, and 0.13 for PS_{0% F127}, PS_{0.0001% F127}, and PS_{0.01% F127}, respectively. The distribution of individual particle speeds shows that PS_{1% F127} exhibited two populations of particles, one consisting of hindered particles with speeds similar to PS_{0% F127}, PS_{0.0001% F127}, and PS_{0.01% F127} and the other consisting of rapidly diffusing particles with speeds similar to the F127-coated PS particles described in Example 2.

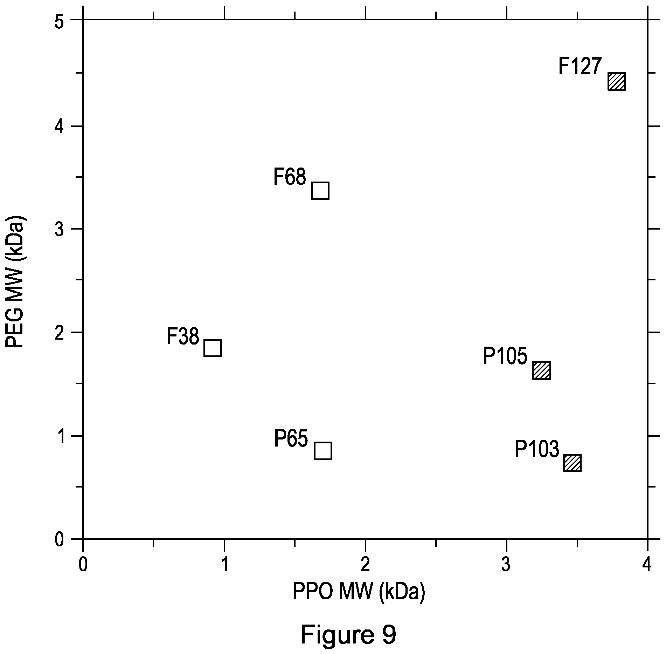

Pluronics® are commercially available in a variety of MW and PPO/PEG segment ratios, and different Pluronics® have been adopted for various biomedical applications. Pluronics®, in addition to F127, that may improve the transport of otherwise mucoadhesive particles upon addition to mucus were investigated. Pluronic® P65, F38, P103, P105, or F68 (listed in order of increasing MW) was added at 1% v/v to human cervicovaginal mucus samples to obtain a final concentration of 0.1% w/v (*i*.*e*., 1 mg/mL) Pluronic®. After addition of Pluronic®, fluorescent 500 nm carboxyl-modified PS particles were added to the mucus samples and incubated at 37 °C for 2 h before microscopy. PS particles added to saline-treated or Pluronic®-treated mucus are referred to as PS, PS_{P65}, PS_{F38}, PS_{P103}, PS_{P105}, or PS_{F68}, respectively. The time-lapse traces of PS_{P65}, PS_{F38} and PS_{F68} were all highly constrained and non-Brownian, while PS_{P103} and PS_{P105} exhibited much more diffusive trajectories over larger distances. Similar to F127, P103- and P105-treatment of mucus improved the <MSD> of PS particles by 25-fold or higher compared to that for PS in saline-treated mucus at a time scale of 1 s. In Figure 9, the mobility of PS particles in fresh human cervicovaginal mucus treated with Pluronic® F68, F38, P65, F127, P103, or P105 is shown. The filled symbols indicate significant improvement of particle transport by Pluronic® treatment, while open symbols indicate little to no insignificant improvements. These results are in good agreement with our findings in Example 2 with Pluronic®-coated particles, where it was demonstrated that F127, P103, and P105 effectively transformed otherwise mucoadhesive particles into mucus-penetrating particles.

### Reference for Example 3:

[1] Lai, S.K., et al., Rapid transport of large polymeric nanoparticles in fresh undiluted human mucus. Proc Natl Acad Sci U S A, 2007. 104(5): p. 1482-7.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, *i.e.,* elements that are conjunctively present in some cases and disjunctively present in other cases. Other elements may optionally be present other than the elements specifically identified by the "and/or" clause, whether related or unrelated to those elements specifically identified unless clearly indicated to the contrary. Thus, as a non-limiting example, a reference to "A and/or B," when used in conjunction with open-ended language such as "comprising" can refer, to A without B (optionally including elements other than B); furthermore, to B without A (optionally including elements other than A); yet further, to both A and B (optionally including other elements); *etc.*

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (*i.e.* "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, to at least one, optionally including more than one, A, with no B present (and optionally including elements other than B); furthermore, to at least one, optionally including more than one, B, with no A present (and optionally including elements other than A); yet further, to at least one, optionally including more than one, A, and at least one, optionally including more than one, B (and optionally including other elements); *etc.*

In the claims, as well as in the specification above, all transitional phrases such as "comprising," "including," "carrying," "having," "containing," "involving," "holding," and the like are to be understood to be open-ended, *i.e.,* to mean including but not limited to. Only the transitional phrases "consisting of' and "consisting essentially of' shall be closed or semi-closed transitional phrases, respectively, as set forth in the United States Patent Office Manual of Patent Examining Procedures, Section 2111.03.

## Claims

1. A method of reducing mucoadhesion of a particle, wherein the particle comprises a poly(ethylene glycol)-vitamin E conjugate associated with at least a portion of the particle, the method comprising the steps of:
associating a (poly(ethylene glycol))-(poly(propylene oxide))-(poly(ethylene glycol)) triblock copolymer with the surface of the particle, thereby forming a coated particle,
wherein the molecular weight of the poly(ethylene glycol) of the poly(ethylene glycol)-vitamin E conjugate is greater than about 2 kDa,
wherein the molecular weight of the (poly(propylene oxide)) block of the triblock copolymer is greater than about 1.8 kDa, and
wherein said coated particle diffuses through human cervicovaginal mucus at a diffusivity that is less than 1/500 the diffusivity that the particle diffuses through water.

2. The method of claim 1, wherein the particle comprises a polymeric material; and the polymeric material is selected from a polyamine, polyether, polyamide, polyester, polycarbamate, polyurea, polycarbonate, poly(styrene), polyimide, polysulfone, polyurethane, polyacetylene, polyethylene, polyethyleneimine, polyisocyanate, polyacrylate, polymethacrylate, polyacrylonitrile, and polyarylate, and wherein the polymeric material is optionally biodegradable and/or biocompatible.

3. The method of claim 1, wherein the particle comprises a hydrophobic material and at least one bioactive agent.

4. The method of claim 1, wherein the molecular weight of the (poly(propylene oxide)) block of the triblock copolymer is between about 1.8 kDa and about 10 kDa, or between about 2 kDa and about 10 kDa, or between about 3 kDa and about 10 kDa, or between about 4 kDa and about 10 kDa, or between about 1.8 kDa and about 5 kDa, or between about 3 kDa and about 5 kDa, or between about 2 kDa and about 4 kDa, or between about 2 kDa and about 5 kDa.

5. The method of claim 1, wherein the molecular weight of the (poly(propylene oxide)) block of the triblock copolymer is at least about 2 kDa, or at least about 2.5 kDa, or at least about 3 kDa, or at least about 4 kDa, or at least about 5 kDa.

6. The method of claim 1, wherein the particle comprises surface-altering moieties disposed on the surface of the particle, wherein the surface-altering moieties optionally comprise regions of the (poly(ethylene glycol))-(poly(propylene oxide))-(poly(ethylene glycol)) triblock copolymer localized on the surface of the particle, and wherein the surface-altering moieties are optionally present at a density between about 0.1 and about 10, or between about 0.1 and about 5, or between about 0.5 and about 5, or between about 0.1 and about 3, or between about 1 and about 10, or between about 0.5 and about 3, or between about 0.9 and about 2.8 surface-altering moieties per nm².

7. The method of claim 1, wherein the particle further comprises at least one bioactive agent, such as an imaging agent, diagnostic agent, therapeutic agent, agent with a detectable label, nucleic acid, nucleic acid analog, small molecule, peptidomimetic, protein, peptide, lipid, or surfactant, and wherein the at least one bioactive agent is optionally encapsulated in the particle and/or is disposed on the surface of the particle.

8. The method of claim 1, wherein the particle is larger than about 1 nm, or about 5 nm, or about 10 nm, or about 20 nm, or about 100 nm, or about 200 nm, or about 500 nm in diameter.

9. A particle comprising:
a poly(ethylene glycol)-vitamin E conjugate; and
a (poly(ethylene glycol))-(poly(propylene oxide))-(poly(ethylene glycol)) triblock copolymer coating,
wherein the molecular weight of the poly(ethylene glycol) of the poly(ethylene glycol)-vitamin E conjugate is greater than about 2 kDa,
wherein the molecular weight of the (poly(propylene oxide)) block of the triblock copolymer is greater than about 1.8 kDa.

10. The particle of claim 9, wherein the molecular weight of the (poly(propylene oxide)) block of the triblock copolymer is between about 1.8 kDa and about 10 kDa, or between about 2 kDa and about 1 0 kDa, or between about 3 kDa and about 1 0 kDa, or between about 4 kDa and about 1 0 kDa, between about 1.8 kDa and about 5 kDa, or between about 3 kDa and about 5 kDa, or between about 2 kDa and about 4 kDa, or between about 2 kDa and about 5 kDa.

11. The particle of claim 9, wherein the molecular weight of the (poly(propylene oxide)) block of the triblock copolymer is at least about 2 kDa, or at least about 2.5 kDa, or at least about 3 kDa, or at least about 4 kDa, or at least about 5 kDa.

12. The particle of claim 9, wherein the particle further comprises at least one bioactive agent, such as an imaging agent, diagnostic agent, therapeutic agent, agent with a detectable label, nucleic acid, nucleic acid analog, small molecule, peptidomimetics, protein, peptide, lipid, or surfactant, and wherein the bioactive agent is optionally encapsulated in the particle and/or is disposed on the surface of the particle, covalently coupled to the particle or is not covalently associated with the particle.

13. The particle of claim 9, wherein the particle is larger than about 1 nm, or about 5 nm, or about 10 nm, or about 20 nm, or about 100 nm, or about 200 nm, or about 500 nm in diameter.

14. A pharmaceutical composition comprising a plurality of particles of any one of claim 9 to 13 and one or more pharmaceutically acceptable excipients.

15. The pharmaceutical composition of claim 14 for use in treating, preventing, or diagnosing a condition in a patient, wherein the pharmaceutical composition is optionally administered to a mucosal tissue in the patient.

## Patentansprüche

1. Verfahren zur Reduktion der Mucoadhäsion eines Partikels, wobei das Partikel ein Poly(ethylenglycol)-Vitamin E-Konjugat, assoziiert mit mindestens einem Teil des Partikels, umfasst, wobei das Verfahren die Schritte umfasst:
Assoziierung eines (Poly(ethylenglycol))-(Poly(propylenoxid))-(Poly(ethylenglycol)) Triblock-Copolymers mit der Oberfläche des Partikels wodurch ein beschichtetes Partikel erhalten wird,
wobei das Molekulargewicht des Poly(ethylenglycols) des Poly(ethylenglycol)-Vitamin E-Konjugats größer als etwa 2 kDa ist,
wobei das Molekulargewicht des (Poly(propylenoxid))-Blocks des Triblock-Copolymers größer als etwa 1,8 kDa ist, und
wobei das beschichtete Partikel durch menschlichen zervixvaginalen Schleim mit einer Diffusität diffundiert, die kleiner als 1/500 der Diffusität ist, mit der das Partikel durch Wasser diffundiert.

2. Verfahren nach Anspruch 1, wobei das Partikel ein polymeres Material umfasst und das polymere Material ausgewählt ist aus einem Polyamin, Polyether, Polyamid, Polyester, Polycarbamat, Polyharnstoff, Polycarbonat, Poly(styren), Polyimid, Polysulfon, Polyurethan, Polyacetylen, Polyethylen, Polyethyleneimin, Polyisocyanat, Polyacrylat, Polymethacrylat, Polyacrylnitril und Polyarylat, und wobei das polymere Material gegebenenfalls biologisch abbaubar und/oder biologisch kompatibel ist.

3. Verfahren nach Anspruch 1, wobei das Partikel ein hydrophobes Material und mindestens ein bioaktives Agens umfasst.

4. Verfahren nach Anspruch 1, wobei das Molekulargewicht des (Poly(propylenoxid))-Blocks des Triblock-Copolymers zwischen etwa 1,8 kDa und etwa 10 kDa, oder zwischen etwa 2 kDa und etwa 10 kDa, oder zwischen etwa 3 kDa und etwa 10 kDa, oder zwischen etwa 4 kDa und etwa 10 kDa, oder zwischen etwa 1,8 kDa und etwa 5 kDa, oder zwischen etwa 3 kDa und etwa 5 kDa, oder zwischen etwa 2 kDa und etwa 4 kDa, oder zwischen etwa 2 kDa und etwa 5 kDa ist.

5. Verfahren nach Anspruch 1, wobei das Molekulargewicht des (Poly(propylenoxid))-Blocks des Triblock-Copolymers mindestens etwa 2 kDa, oder mindestens etwa 2,5 kDa, oder mindestens etwa 3 kDa, oder mindestens etwa 4 kDa, oder mindestens etwa 5 kDa ist.

6. Verfahren nach Anspruch 1, wobei das Partikel oberflächenverändernde Einheiten, gelegen auf der Oberfläche des Partikels umfasst, wobei die oberflächenverändernden Einheiten gegebenenfalls Regionen des (Poly(ethylenglycol))-(Poly(propylenoxid))-(Poly(ethylenglycol))-Triblock-Copolymers, das auf der Oberfläche des Partikels lokalisiert ist, umfasst und wobei die oberflächenverändernden Einheiten gegebenenfalls in einer Dichte zwischen etwa 0,1 und etwa 10, oder zwischen etwa 0,1 und etwa 5, oder zwischen etwa 0,5 und etwa 5, oder zwischen etwa 0,1 und etwa 3, oder zwischen etwa 1 und etwa 10, oder zwischen etwa 0,5 und etwa 3, oder zwischen etwa 0,9 und etwa 2,8 oberflächenverändernde Einheiten pro nm² anwesend sind.

7. Verfahren nach Anspruch 1, wobei das Partikel weiterhin mindestens ein bioaktives Agens umfasst, wie etwa ein/e bildgebendes Agens, diagnostisches Agens, therapeutisches Agens, Agens mit einer nachweisbaren Markierung, Nukleinsäure, Nukleinsäureanalog, kleines Molekül, Peptidomimetik, Protein, Peptid, Lipid oder oberflächenaktiver Stoff, und wobei das mindestens eine bioaktive Agens gegebenenfalls in dem Partikel eingeschlossen ist und/oder auf der Oberfläche des Partikels angeordnet ist.

8. Verfahren nach Anspruch 1, wobei das Partikel im Durchmesser größer ist als etwa 1 nm, oder etwa 5 nm, oder etwa 10 nm, oder etwa 20 nm, oder etwa 100 nm, oder etwa 200 nm, oder etwa 500 nm.

9. Partikel, umfassend:
ein Poly(ethylenglycol)-Vitamin E-Konjugat, und
eine (Poly(ethylenglycol))-(Poly(propylenoxid))-(Poly(ethylenglycol))-Triblock-Copolymer-Beschichtung,
wobei das Molekulargewicht des Poly(ethylenglycols) des Poly(ethylenglycol)-Vitamin E-Konjugats größer als etwa 2 kDa ist,
wobei das Molekulargewicht des (Poly(propylenoxid))-Blocks des Triblock-Copolymers größer als etwa 1,8 kDa ist.

10. Partikel nach Anspruch 9, wobei das Molekulargewicht des (Poly(propylenoxid))-Blocks des Triblock-Copolymers zwischen etwa 1,8 kDa und etwa 10 kDa, oder zwischen etwa 2 kDa und etwa 10 kDa, oder zwischen etwa 3 kDa und etwa 10 kDa, oder zwischen etwa 4 kDa und etwa 10 kDa, zwischen etwa 1,8 kDa und etwa 5 kDa, oder zwischen etwa 3 kDa und etwa 5 kDa, oder zwischen etwa 2 kDa und etwa 4 kDa, oder zwischen etwa 2 kDa und etwa 5 kDa ist.

11. Partikel nach Anspruch 9, wobei das Molekulargewicht des (Poly(propylenoxid))-Blocks des Triblock-Copolymers mindestens etwa 2 kDa, oder mindestens etwa 2,5 kDa, oder mindestens etwa 3 kDa, oder mindestens etwa 4 kDa, oder mindestens etwa 5 kDa ist.

12. Partikel nach Anspruch 9, wobei das Partikel weiterhin mindestens ein bioaktives Agens umfasst, wie etwa ein/e bildgebendes Agens, diagnostisches Agens, therapeutisches Agens, Agens mit einer nachweisbaren Markierung, Nukleinsäure, Nukleinsäureanalog, kleines Molekül, Peptidomimetik, Protein, Peptid, Lipid, oder oberflächenaktiver Stoff, und wobei das bioaktive Agens gegebenenfalls in dem Partikel eingeschlossen ist und/oder auf der Oberfläche des Partikels angeordnet ist, kovalent mit dem Partikel verbunden ist oder nicht kovalent mit dem Partikel assoziiert ist.

13. Partikel nach Anspruch 9, wobei das Partikel im Durchmesser größer ist als etwa 1 nm, oder etwa 5 nm, oder etwa 10 nm, oder etwa 20 nm, oder etwa 100 nm, oder etwa 200 nm oder etwa 500 nm.

14. Pharmazeutische Zusammensetzung, umfassend eine Vielzahl von Partikeln nach einem der Ansprüche 9 bis 13 und einen oder mehrere pharmazeutische Hilfsstoffe.

15. Pharmazeutische Zusammensetzung nach Anspruch 14 zur Verwendung in der Behandlung, Prävention oder Diagnose einer Erkrankung in einem Patienten, wobei die pharmazeutische Zusammensetzung gegebenenfalls an ein Schleimhautgewebe in dem Patienten verabreicht wird.

## Revendications

1. Méthode de réduction de la mucoadhésion d'une particule, dans laquelle la particule comprend un conjugué poly(éthylène glycol)-vitamine E associé à au moins une partie de la particule, la méthode comprenant les étapes de :
association d'un copolymère tribloc (poly(éthylène glycol))-(poly(oxyde de propylène))-(poly(éthylène glycol)) à la surface de la particule, pour former ainsi une particule revêtue,
dans laquelle le poids moléculaire du poly(éthylène glycol) du conjugué poly(éthylène glycol)-vitamine E est supérieur à environ 2 kDa,
dans laquelle le poids moléculaire du bloc (poly(oxyde de propylène)) du copolymère tribloc est supérieur à environ 1,8 kDa, et
dans laquelle ladite particule revêtue se diffuse dans le mucus cervicovaginal humain à une diffusivité qui est inférieure à 1/500 de la diffusivité à laquelle la particule se diffuse dans l'eau.

2. Méthode selon la revendication 1, dans laquelle la particule comprend un matériau polymère ; et le matériau polymère est choisi parmi une polyamine, un polyéther, un polyamide, un polyester, un polycarbamate, une polyurée, un polycarbonate, un poly(styrène), un polyimide, une polysulfone, un polyuréthane, un polyacétylène, un polyéthylène, un polyéthylèneimine, un polyisocyanate, un polyacrylate, un polyméthacrylate, un polyacrylonitrile, et un polyarylate, et dans laquelle le matériau polymère est éventuellement biodégradable et/ou biocompatible.

3. Méthode selon la revendication 1, dans laquelle la particule comprend un matériau hydrophobe et au moins un agent bioactif.

4. Méthode selon la revendication 1, dans laquelle le poids moléculaire du bloc (poly(oxyde de propylène)) du copolymère tribloc se situe entre environ 1,8 kDa et environ 10 kDa, ou entre environ 2 kDa et environ 10 kDa, ou entre environ 3 kDa et environ 10 kDa, ou entre environ 4 kDa et environ 10 kDa, ou entre environ 1,8 kDa et environ 5 kDa, ou entre environ 3 kDa et environ 5 kDa, ou entre environ 2 kDa et environ 4 kDa, ou entre environ 2 kDa et environ 5 kDa.

5. Méthode selon la revendication 1, dans laquelle le poids moléculaire du bloc (poly(oxyde de propylène)) du copolymère tribloc est d'au moins environ 2 kDa, ou d'au moins environ 2,5 kDa, ou d'au moins environ 3 kDa, ou d'au moins environ 4 kDa, ou d'au moins environ 5 kDa.

6. Méthode selon la revendication 1, dans laquelle la particule comprend des fragments altérant la surface placés sur la surface de la particule, dans laquelle les fragments altérant la surface comprennent éventuellement des régions du copolymère tribloc (poly(éthylène glycol))-(poly(oxyde de propylène))-(poly(éthylène glycol)) situé à la surface de la particule, et dans laquelle les fragments altérant la surface sont éventuellement présents à une densité comprise entre environ 0,1 et environ 10, ou entre environ 0,1 et environ 5, ou entre environ 0,5 et environ 5, ou entre environ 0,1 et environ 3, ou entre environ 1 et environ 10, ou entre environ 0,5 et environ 3, ou entre environ 0,9 et environ 2,8 fragments altérant la surface par nm².

7. Méthode selon la revendication 1, dans laquelle la particule comprend en outre au moins un agent bioactif, tel qu'un agent d'imagerie, un agent diagnostique, un agent thérapeutique, un agent pourvu d'un marqueur détectable, un acide nucléique, un analogue d'acide nucléique, une petite molécule, un peptidomimétique, une protéine, un peptide, un lipide, ou un tensioactif, et dans laquelle ledit au moins agent bioactif est éventuellement encapsulé dans la particule et/ou est disposé sur la surface de la particule.

8. Méthode selon la revendication 1, dans laquelle la particule a un diamètre supérieur à environ 1 nm, ou environ 5 nm, ou environ 10 nm, ou environ 20 nm, ou environ 100 nm, ou environ 200 nm, ou environ 500 nm.

9. Particule comprenant :
un conjugué poly(éthylène glycol)-vitamine E ; et
un revêtement à base d'un copolymère tribloc (poly(éthylène glycol))-(poly(oxyde de propylène))-(poly(éthylène glycol)),
dans laquelle le poids moléculaire du poly(éthylène glycol) du conjugué poly(éthylène glycol)-vitamine E est supérieur à environ 2 kDa,
dans laquelle le poids moléculaire du bloc (poly(oxyde de propylène)) du copolymère tribloc est supérieur à environ 1,8 kDa.

10. Particule selon la revendication 9, dans laquelle le poids moléculaire du bloc (poly(oxyde de propylène)) du copolymère tribloc se situe entre environ 1,8 kDa et environ 10 kDa, ou entre environ 2 kDa et environ 10 kDa, ou entre environ 3 kDa et environ 10 kDa, ou entre environ 4 kDa et environ 10 kDa, entre environ 1,8 kDa et environ 5 kDa, ou entre environ 3 kDa et environ 5 kDa, ou entre environ 2 kDa et environ 4 kDa, ou entre environ 2 kDa et environ 5 kDa.

11. Particule selon la revendication 9, dans laquelle le poids moléculaire du bloc (poly(oxyde de propylène)) du copolymère tribloc est d'au moins environ 2 kDa, ou d'au moins environ 2,5 kDa, ou d'au moins environ 3 kDa, ou d'au moins environ 4 kDa, ou d'au moins environ 5 kDa.

12. Particule selon la revendication 9, dans laquelle la particule comprend en outre au moins un agent bioactif, tel qu'un agent d'imagerie, un agent diagnostique, un agent thérapeutique, un agent pourvu d'un marqueur détectable, un acide nucléique, un analogue d'acide nucléique, une petite molécule, un peptidomimétique, une protéine, un peptide, un lipide, ou un tensioactif, et dans laquelle ledit au moins agent bioactif est éventuellement encapsulé dans la particule et/ou est disposé sur la surface de la particule, couplé de manière covalente à la particule ou n'est pas associé de manière covalente à la particule.

13. Particule selon la revendication 9, dans laquelle la particule a un diamètre supérieur à environ 1 nm, ou environ 5 nm, ou environ 10 nm, ou environ 20 nm, ou environ 100 nm, ou environ 200 nm, ou environ 500 nm.

14. Composition pharmaceutique comprenant une pluralité de particules selon l'une quelconque des revendications 9 à 13 et un ou plusieurs excipients pharmaceutiquement acceptables.

15. Composition pharmaceutique selon la revendication 14 pour une utilisation dans le traitement, la prévention, ou le diagnostic d'une affection chez un patient, dans laquelle la composition pharmaceutique est éventuellement administrée au niveau d'un tissu muqueux du patient.
